# EUROPEAN PATENT SPECIFICATION

(11) **EP 2 343 059 B1**
(45) Date of publication and mention of the grant of the patent: **02.04.2014**
(21) Application number: 09816071.6
(22) Date of filing: 15.09.2009
(51) Int. Cl.: A61K 9/24, A61K 9/48, A61K 47/32, A61K 47/38, A61K 9/20, A61K 9/28

(54) **ORAL PREPARATION**
ORALE ZUBEREITUNG
PRÉPARATION ORALE

(30) Priority: 29.09.2008 JP 2008251786
(43) Date of publication of application: 13.07.2011
(73) Proprietor: LINTEC Corporation, Tokyo 173-0001 (JP)
(72) Inventor: TAKAHASHI Akira, Tokyo 173-0001 (JP); SUGIURA Yusaku, Tokyo 173-0001 (JP); HIRONAGA Maki, Tokyo 173-0001 (JP); KABUTO Akio, Tokyo 173-0001 (JP); SUZUKI Eiji, Tokyo 173-0001 (JP)
(74) Representative: Solf, Alexander
(86) International application number: PCT/JP2009/066066
(87) International publication number: WO 2010/035656

(56) References cited:
- WO-A1-02/087622
- WO-A1-2005/087205
- WO-A1-2005/097080
- WO-A1-2005/097198
- WO-A1-2008/126488
- WO-A1-2008/129730
- WO-A1-2008/129730
- WO-A1-2009/072572
- JP-A- 1 193 215
- JP-A- 59 051 223
- JP-A- 2004 248 665
- US-A- 4 765 983
- US-A- 4 889 720
- US-A1- 2007 141 152

## Description

### TECHNICAL FIELD

The present invention relates to an orally-administered agent.

### RELATED ART

As examples of an orally-administered agent containing a medicine, there are known solid formulations and jelly-like (or gel-like) semisolid formulations. The solid formulations (e.g., tablets and capsules) are usually hard to take as they are and therefore have to be taken together with a large quantity of water. Thus, it is often difficult for aged persons or infants to take the solid formulations. In addition, there are risks that the solid formulations are likely to get stuck in a trachea or may adhere to an esophagus.

In contrast, the jelly-like semisolid formulations are easy to swallow. Therefore, the jelly-like semisolid formulations can be easily taken by even aged persons or infants. However, since the semisolid formulations contain a large quantity of moisture, they have a drawback in that the medicine contained therein is susceptible to decomposition or degradation. Moreover, there is a need that the semisolid formulations prevent infiltration of bacteria when producing and storing the semisolid formulations. This makes it cumbersome and complicated to handle the semisolid formulations. For these reasons stated above, such drawbacks prevent the semisolid formulations from being widespread in a market.

In view of the problems noted above, a study has been made in recent years on thin sheet-like (film-like) formulations (see, e.g., the following Patent Document). Within an oral cavity, the film-like formulations are dissolved by saliva or the film-like formulations are gelatinized by absorbing water. Therefore, it is relatively easy to swallow the film-like formulations. In addition, there is no need to add water into the film-like formulations. This makes it easy to handle the film-like formulations when producing and storing the same.

However, since the film-like formulations are relatively thin sheet-like formulations, a medicine contained therein is likely to flow out by body fluids such as saliva. Therefore, in the case where such film-like formulations are used, it is difficult to release a medicine at intended organs of a living body (e.g. intestines and stomach) and make the medicine absorbed into the organs. Furthermore, in the case where the medicine contained in the film-like formulations flows out into the oral cavity by saliva, there is a case that the medicine may give unpleasant feelings to recipients due to tastes which the medicine itself has (e.g., a bitter taste and an astringent taste), senses within the oral cavity by the medicine (e.g., a sense of numbness), odors of the medicine and the like. Therefore, there is a problem in that it is difficult for patients to follow the compliance of the medicine.

Furthermore, in the case where a medicine to be decomposed by gastric acid is made to be absorbed from an intestinal tract, the following means are performed in order to prevent such a medicine from being decomposed by the gastric acid thereby exhibiting no medicinal benefits: the medicine is coated with an enteric material to obtain an enteric coated medicine after such a medicine is processed in tablet form; a granular material of such a medicine is filled in an enteric capsule; and the like. However, it is difficult to swallow such an enteric coated medicine and the like without water.

The Patent Document is JP-A 11-116469 as an example of related art.

The document US 2007141152 discloses a multilayer composition for oral administration. The most external layer comprises an acrylate polymer (gel). The middle layer comprises PVP (adhesive), and the layer inside comprises a drug.

### SUMMARY OF THE INVENTION

It is an object of the present invention to provide an orally-administered agent that can be swallowed with ease and can release a medicine at intended parts of a living body (in particular, within intestines).

Such an object is achieved by the present invention which is described below:
a) An orally-administered agent (1b;1c;1d;1e) comprising:
   a medicine-containing layer, (11;11a) containing a medicine and having surfaces;
   two intestinal collapse-controlling layers (12a;12b;12c;12d) including an enteric material which is dissolved by being in contact with the intestinal body fluid, wherein the intestinal collapse-controlling layers (12a;12b;12c;12d) are provided directly or through an arbitrary layer on the surfaces of the medicine-containing layer (11;11a), respectively, and each of the intestinal collapse-controlling layers (12a;12b;12c;12d) has a surface opposite to the medicine-containing layer (11;11a); and
   two gel-forming layers (13c;13d;13e;13f) containing a gel- forming agent that is swelled and gelatinized by absorbing water, wherein the gel-forming layers (13c;13d;13e;13f) are provided directly or through an arbitrary layer on the sides of the surfaces of the intestinal collapse-controlling layers (12a;12b;12c;12d), respectively,
   wherein each of the gel-forming layers (13c;13d;13e;13f) has a surface provided with a plurality of convex portions and a pitch p between the convex portions is in a range of 100 to 1,000 µm, and
   wherein the surfaces of the gel-forming layers (13c;13d;13e;13f) define outer surfaces of the orally-administered agent (1b;1c;1d;1e).
b) An orally-administered agent (1a) comprising:
   a medicine-containing layer (11) containing a medicine and having surfaces;
   two intestinal collapse-controlling layers (12a; 12b) including an enteric material which is dissolved by being in contact with the intestinal body fluid wherein the intestinal collapse-controlling layers (12a;12b) are provided directly or through an arbitrary layer on the surfaces of the medicine-containing layer (11), respectively, and each of the intestinal collapse-controlling layers (12a;12b) has a surface opposite to the medicine-containing layer (11); and
   two gel-forming layers (13a, 13b) containing a gel-forming agent that is swelled and gelatinized by absorbing water, wherein the gel-forming layers (13a, 13b) are provided directly or through an arbitrary layer on the sides of the surfaces of the intestinal collapse-controlling layers (12a;12b), respectively;
   two antiadhesive layers (14a;14b) including an antiadhesive agent as surface layers constituting surfaces of the orally-administered agent (1a),
   wherein a viscosity at 37°C of an aqueous solution of 5 mass% of the antiadhesive agent measured with an E-type viscometer is 50 mPa*s or less
   wherein the antiadhesive layers (14a;14b) prevent the orally administered agent (1a) from adhering to an inside wall of an oral cavity by dissolving to water.

According to the present invention, it is possible to provide an orally-administered agent that can be swallowed with ease and can release a medicine at intended parts of a living body (in particular, within intestines).

### BRIEF DESCRIPTION OF THE DRAWINGS

FIG. 1 is a section view showing an orally-administered agent in accordance with a first embodiment of the present invention.
FIG. 2 is a section view showing an orally-administered agent in accordance with a second embodiment of the present invention.
FIG. 3 is a section view showing an orally-administered agent in accordance with a third embodiment of the present invention.
FIG. 4 is a section view showing an orally-administered agent in accordance with a fourth embodiment of the present invention.
FIG. 5 is a section view showing an orally-administered agent in accordance with a fifth embodiment of the present invention.

### DETAILED DESCRIPTION OF THE PREFERRED EMBODIMENTS

Hereinafter, the present invention will be described in detail based on certain preferred embodiments.

An orally-administered agent of the present invention may have any shape. The following description will be made on the assumption that the present orally-administered agent is a film-like formulation (or a sheet-like formulation).

Hereinbelow, embodiments of the present invention will be described with reference to the accompanying drawings.

### <First Embodiment>

First, a description will be made on an orally-administered agent in accordance with a first embodiment of the present invention.

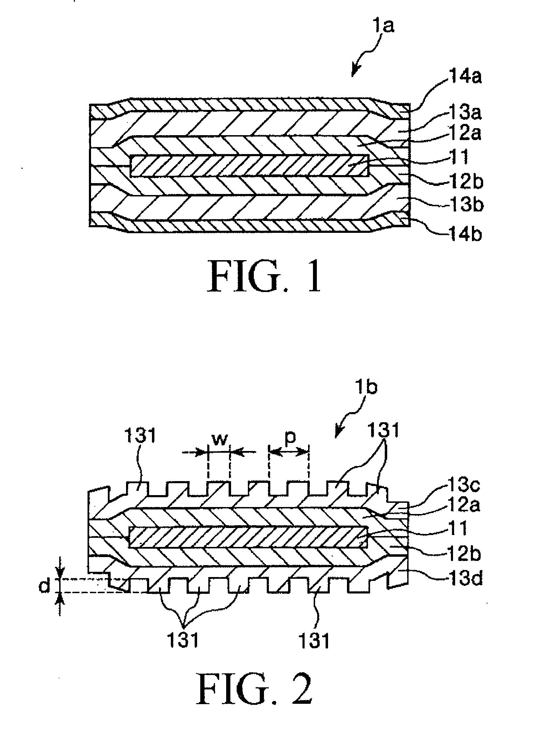

FIG. 1 is a section view showing an orally-administered agent in accordance with a first embodiment of the present invention. In the following description, the upper side in FIG. 1 will be referred to as "upper" and the lower side thereof will be referred to as "lower" for convenience of explanation.

As shown in FIG. 1, the orally-administered agent 1a is configured as a laminated body. Such a laminated body includes an intestinal medicine-containing layer 11 which contains a medicine, an intestinal collapse-controlling layer 12a which is laminated on an upper surface of the intestinal medicine-containing layer 11, an intestinal collapse-controlling layer 12b which is laminated on a lower surface of the intestinal medicine-containing layer 11, a gel-forming layer 13a which is laminated on an upper surface of the intestinal collapse-controlling layer 12a, and a gel-forming layer 13b which is laminated on a lower surface of the intestinal collapse-controlling layer 12b. That is, the intestinal medicine-containing layer 11 is completely covered with the intestinal collapse-controlling layers 12a and 12b.

The orally-administered agent 1a is a film-like formulation (or a sheet-like formulation) in a whole shape thereof. Since the orally-administered agent 1a is the film-like formulation, it is possible to reduce a moisture content in the film-like formulation. Furthermore, it is possible to enhance stability of the medicine (especially, an easily-hydrolysable medicine) contained in the intestinal medicine-containing layer 11 as compared with a jelly-like formulation which contains a large quantity of moisture. Moreover, the film-like formulation is easy to handle, and it is possible to assist in reducing a packing cost of the film-like formulation.

Furthermore, the gel-forming layers 13a and 13b of the orally-administered agent 1a can be swelled and gelatinized within the oral cavity of a patient by water contained in saliva etc. This makes it possible to change a state of the orally-administered agent 1a to a state of it having a size, a shape, elastic force, a viscosity and the like for allowing a patient to swallow it with ease. Accordingly, the patient can easily take the orally-administered agent 1a. Furthermore, the orally-administered agent 1a has a low risk of getting stuck in a trachea of the patient when swallowing the orally-administered agent 1a. Therefore, even in the case where the patient is aged persons or infants, it is possible to take the orally-administered agent 1a safely.

In addition, the orally-administered agent 1a includes the intestinal collapse-controlling layers 12a and 12b between the intestinal medicine-containing layer 11 and the gel-forming layers 13a and 13b, respectively. The present invention has one of features in that the orally-administered agent 1a includes the intestinal collapse-controlling layers 12a and 12b. The intestinal collapse-controlling layers 12a and 12b include an enteric material which is dissolved by being in contact with a body fluid within intestines. Accordingly, the intestinal collapse-controlling layers 12a and 12b are collapsed by being in contact with the intestinal body fluid in the intestines. As a result, the intestinal medicine-containing layer 11 can be dissolved by being in contact with the body fluid within the intestines, thereby releasing the medicine contained in the intestinal medicine-containing layer 11 into the intestines.

Furthermore, as shown in FIG. 1, it is preferred that the laminated body further includes an antiadhesive layer 14a which is provided on an upper surface of the gel-forming layer 13a and an antiadhesive layer 14b which is provided on a lower surface of the gel-forming layer 13b as surface layers of the orally-administered agent 1a. The antiadhesive layers 14a and 14b are rapidly dissolved by water contained in saliva within the oral cavity and have a function of preventing the orally-administered agent 1a from adhering to the inside wall in the oral cavity.

In view of the above, the orally-administered agent 1a exhibits excellent swallowability and can release the medicine within the intestines.

Hereinbelow, the respective layers constituting the orally-administered agent 1a will be described in more detail.

### <Intestinal Medicine-Containing Layer>

The intestinal medicine-containing layer 11 is a layer containing the medicine to be administered into the intended parts of the living body.

The medicine contained in the intestinal medicine-containing layer 11 is a medicine to be administered to the intended parts of the living body of a patient. Such a medicine is not limited to a specific one but may be any orally-administrable medicine. Examples of the orally-administrable medicine include: medicines acting on a central nerve, including a hypnotic medicine such as amobarbital, estazoram, triazolam, nitrazepam, pentobarbital or the like, a psychotropic medicine such as amitriptyline hydrochloride, imipramine hydrochloride, oxazolam, chlordiazepoxide, chlorpromazine, diazepam, sulpiride, haloperidol or the like, an antiparkinson medicine such as trihexyphenidyl, levodopa or the like, an analgesic medicine and an anti-inflammatory medicine such as aspirin, isopropylantipyrine, indometacin, diclofenac sodium, mefenamic acid, streptokinase, streptodornase, serrapeptase, pronase or the like and a central nervous metabolic activation medicine such as ATP, vinpocetine or the like; medicines acting on a respiratory organ, including an expectorant medicine such as carbocysteine, bromhexine hydrochloride or the like and an antiasthmatic medicine such as azelastine hydrochloride, oxatomide, theophylline, terbutaline sulfate, tranilast, procaterol hydrochloride, ketotifen fumarate or the like; medicines acting on a circulatory system, including a cardiac stimulant such as aminophylline, digitoxin, digoxin or the like, an antiarrhythmic medicine such as ajmaline, disopyramide, procainamide hydrochloride, mexiletine hydrochloride or the like, an antianginal medicine such as amyl nitrite, alprenolol hydrochloride, isosorbide dinitrate, nicorandil, oxyfedrine, dipyridamole, dilazep hydrochloride, diltiazem hydrochloride, nitroglycerin, nifedipine, verapamil hydrochloride or the like, a peripheral vasodilator such as kallidinogenase or the like, an antihypertensive medicine such as atenolol, captopril, clonidine hydrochloride, metoprolol tartrate, spironolactone, triamterene, trichlormethiazide, nicardipine, hydralazine hydrochloride, hydrochlorothiazide, prazosin hydrochloride, furosemide, propranolol hydrochloride, enalapril maleate, methyldopa, labetalol hydrochloride, reserpine or the like and an antiarteriosclerotic medicine such as clofibrat, dextran sulfate, nicomol, niceritrol or the like; blood and hematopoietic medicines, including a hemostatic medicine such as carbazochrome sodium sulfonate, tranexamic acid or the like, an antithrombogenic medicine such as ticlopidine hydrochloride, warfarin potassium or the like and an anemia medicine such as ferric sulfate or the like; medicines acting on a gastrointestinal system, including an antiulcer medicine such as azulene, aldioxa, cimetidine, ranitidine hydrochloride, famotidine, teprenone, rebamipide or the like, an antiemetic medicine such as domperidone, metoclopramide or the like, a cathartic medicine such as sennoside, digestive enzyme preparations, and a therapeutic medicine for liver diseases such as glycyrrhizin, liver extract preparations or the like; medicines acting on a metabolic disease, including an antidiabetic medicine such as glibenclamide, chlorpropamide, tolbutamide or the like and an antipodagric medicine such as allopurinol, colchicines or the like; medicines for an ophthalmic field, including acetazolamide; medicines for an otological field, including an anti-vertigo medicine such as difenidol hydrochloride, betahistine mesylate or the like; chemotherapeutic medicines and antibiotic medicines including isoniazid, ethambutol hydrochloride, ofloxacin, erythromycin stearate, cefaclor, norfloxacin, fosfomycin calcium, minocycline hydrochloride, rifampicin, rokitamycin or the like; antineoplastic medicines including cyclophosphamide, tegafur or the like; immunosuppressive medicines including azathioprine; hormones and endocrine medicines including progestational hormone, salivary hormone, thiamazole, prednisolone, betamethasone, liothyronine, levothyroxine or the like; and physiologically active substances (autacoids) including an antihistamine medicine such as diphenhydramine hydrochloride, clemastine fumarate, D-chlorpheniramine maleate or the like and a vitamin such as alfacalcidol, cobamamide, tocopherol nicotinate, mecobalamin or the like. One or more of these medicines may be used independently or in combination according to the purposes of treatment and prevention of a condition.

In particular, in the present invention, the orally-administered agent 1a can release the medicine within the intestines. Therefore, it is preferred that used is a medicine of generating effects by being absorbed in the intestines as the medicine contained in the intestinal medicine-containing layer 11.

Furthermore, various kinds of medicines including a medicine administered in a small quantity and a medicine administered in a large quantity can be contained in the intestinal medicine-containing layer 11. In this regard, the medicine administered in a small quantity means a medicine whose one-time dosage amount is 1 mg or less, while the medicine administered in a large quantity means a medicine whose one-time dosage amount is 300 mg or more.

A content of the medicine in the intestinal medicine-containing layer 11 is not particularly limited and may be suitably adjusted depending on a kind of medicine and the volume of the intestinal medicine-containing layer 11. The content of the medicine is preferably in the range of 0.01 to 70 mass%, more preferably in the range of 0.01 to 40 mass% and even more preferably in the range of 0.01 to 35 mass%. This makes it possible to have a sufficiently quantity of the medicine contained in the orally-administered agent 1a while enhancing physical strength of the orally-administered agent 1a.

Furthermore, the orally-administered agent 1a exhibits great enough physical strength even when a relatively large quantity of the medicine as described above is contained in the intestinal medicine-containing layer 11 or when an insoluble to water and bulky medicine having a tendency to reduce the physical strength of the intestinal medicine-containing layer 11 is contained in the intestinal medicine-containing layer 11. Presumably, this is because the intestinal collapse-controlling layers 12a and 12b impart great enough physical strength to the orally-administered agent 1a by providing the intestinal collapse-controlling layer 12a provided on the upper surface of the intestinal medicine-containing layer 11 and the intestinal collapse-controlling layer 12b provided on the lower surface thereof in the orally-administered agent 1a.

The intestinal medicine-containing layer 11 may include a base (namely, a base agent for the intestinal medicine-containing layer) which serves to keep the administered medicine to the intended parts of the living body in a desired state in the intestinal medicine-containing layer 11 and to adjust the shape and the physical strength of the intestinal medicine-containing layer 11. Examples of the base used in the intestinal medicine-containing layer 11 include, but are not limited to: cellulose such as crystalline cellulose, carboxymethyl cellulose, hydroxypropyl cellulose, hydroxypropyl methyl cellulose, methyl cellulose, ethyl cellulose, acetyl cellulose, cellulose acetate phthalate, hydroxypropylmethyl cellulose phthalate, hydroxypropylmethyl cellulose acetate sucinate and carboxymethylethyl cellulose; derivatives of cellulose or pharmaceutically acceptable salts of cellulose (e.g., sodium salt); starch such as α-starch, oxidized starch, carboxymethyl starch sodium, hydroxypropyl starch, dextrin and dextran; derivatives of starch; saccharides such as saccharose, maltose, lactose, glucose, fructose, pullulan, xanthane gum, cyclodextrin, xylitol, mannitol and sorbitol; acrylic-acid derivatives such as a dimethylaminoethyl (metha)acrylate-(metha)acrylic acid copolymer, a (metha)acrylic acid-ethylacrylate copolymer, a (metha)acrylic acid-methyl(metha)acrylate copolymer, an ethyl(metha)acrylate-chlorotrimethylammonium (metha)acrylate copolymer, a dimethylaminoethyl (metha)acrylate-chloromethyl (metha)acrylate copolymer and a (metha)acrylic acid-chloroethyl acrylate copolymer; Sellac; polyvinylacetal diethylamino acetate; polyvinyl acetate; polyvinyl alcohol; polyvinyl pyrrolidone; a vinylacetate-vinylpyrrolidone copolymer; natural rubbers such as Arabic gum and tragacanth gum; polyglucosamines such as chitin and chitosan; proteins such as gelatin, casein and soybean protein; titanium oxide; calcium monohydrogen phosphate; calcium carbonate; talc; stearate; magnesium aluminometasilicate; magnesium silicate; and silicic anhydride. One or more of these bases may be used independently or in combination according to the purposes of including the base into the intestinal medicine-containing layer 11.

A content of the base in the intestinal medicine-containing layer 11 is not particularly limited, but may be preferably in the range of 30 to 99.9 mass%, more preferably in the range of 60 to 99.9 mass% and even more preferably in the range of 65 to 99.0 mass%. This makes it possible to sufficiently enhance the physical strength of the intestinal medicine-containing layer 11 with ease while allowing a sufficiently quantity of the medicine to be contained in the intestinal medicine-containing layer 11.

A thickness of the intestinal medicine-containing layer 11 can be suitably adjusted within a range permitting an oral administration of the orally-administered agent 1a. The thickness of the intestinal medicine-containing layer 11 is not particularly limited, but may be preferably in the range of 0.5 to 5000 µm, more preferably in the range of 10 to 3000 µm and even more preferably in the range of 50 to 1000 µm. This makes it possible to sharply reduce variations in the medicine content and the thickness which would occur in respective portions of the intestinal medicine-containing layer 11. In addition, this makes it possible to sufficiently increase overall softness of the orally-administered agent 1a and to greatly enhance ease of swallowing the orally-administered agent 1a.

### <Intestinal Collapse-Controlling Layer>

The intestinal collapse-controlling layer 12a is provided between the intestinal medicine-containing layer 11 and the gel-forming layer 13a. Furthermore, the intestinal collapse-controlling layer 12b is provided between the intestinal medicine-containing layer 11 and the gel-forming layer 13b.

In addition, the intestinal collapse-controlling layers 12a and 12b are bonded to each other so as to cover the intestinal medicine-containing layer 11. Therefore, the circumference of the intestinal medicine-containing layer 11 is covered by the intestinal collapse-controlling layers 12a and 12b. This makes it possible for the intestinal medicine-containing layer 11 to prevent the medicine contained therein from inadvertently being eluted and altered by being in contact with the body fluids carelessly.

Each of the intestinal collapse-controlling layers 12a and 12b are a layer of being capable of collapsing or dissolving by being in contact with the intestinal body fluid in the intestines. Therefore, the intestinal collapse-controlling layers 12a and 12b are collapsed within the intestines, thereby enabling the intestinal medicine-containing layer 11 to be dissolved by being in contact with the body fluids. Consequently, the medicine is released into the intestines.

Furthermore, the intestinal collapse-controlling layers 12a and 12b of the orally-administered agent 1a also have a function of preventing contact between the intestinal medicine-containing layer 11 and the body fluid such as saliva and a gastric juice. Therefore, the intestinal collapse-controlling layers 12a and 12b can prevent the medicine contained in the intestinal medicine-containing layer 11 from being dissolved into the oral cavity. This makes it possible to mask a taste of the medicine (e.g. bitter taste and astringent taste), a sense of the oral cavity generated by the medicine (numbness) or an odor of the medicine. Furthermore, it is possible to prevent an unintended medicine from being released into the stomach.

Hereinafter, since the intestinal collapse-controlling layers 12a and 12b have the same configuration, a description will be made on the intestinal collapse-controlling layer 12a as a representative.

Furthermore, the intestinal collapse-controlling layer 12a include an enteric material which is dissolved by being in contact with the intestinal body fluid. This makes it possible to reliably collapse the intestinal collapse-controlling layer 12a in the intestines. By including such an enteric material, the intestinal collapse-controlling layer 12a is in contact with the body fluid within the intestines and then collapsed or dissolved.

In this regard, the enteric material, for example, corresponds to a material being "practically insoluble" in water in the conditions defined in Japanese Pharmacopoeia Fifteenth Edition, and is a material of dissolving to an alkali solution. Concretely, examples of the enteric material include an intestinal polymer and the like. The intestinal polymer is a component of contributing to maintain a shape of the intestinal collapse-controlling layer 12a when storing the orally-administered agent 1a. Furthermore, the intestinal polymer is a component of being capable of dissolving by reliably being in contact with the body fluid in the intestines.

The enteric material, which can be used in the intestinal collapse-controlling layer 12a, is not particularly limited. For example, examples of the enteric material include: an enteric cellulose derivative, an enteric acrylic acid-based copolymer, an enteric maleic acid-based copolymer, an enteric polyvinyl derivative, shellac and the like. One or more of these compounds may be used independently or in combination.

Examples of such an enteric cellulose derivative include: hydroxy-propyl-methyl cellulose acetate succinate, hydroxy-propyl-methyl cellulose phthalate, hydroxy-propyl-methyl acetate maleate, hydroxy-methyl-ethyl cellulose phthalate, cellulose acetate phthalate, cellulose acetate succinate, cellulose acetate trimellitate, cellulose acetate maleate, cellulose benzoate phthalate, cellulose propionate phthalate, methyl cellulose phthalate, ethyl-hydroxy-ethyl cellulose phthalate, hydroxy-methyl cellulose ethylphthalate, cellulose acetate phthalate and the like.

Examples of the enteric acrylic acid-based copolymer include: a methacrylic acid-methyl methacrylate copolymer, methacrylic acid-ethyl acrylate, a styrene-acrylic acid copolymer, a methyl acrylate-acrylic acid copolymer, a methyl acrylate-methacrylic acid copolymer, a buthyl acrylate-acrylatestyrene-acrylic acid copolymer, a methyl acrylate-methacrylic acid-octyl acrylate copolymer and the like.

Examples of the enteric maleic acid-based copolymer include: a vinyl acetate-maleic anhydride copolymer, a styrene-maleic anhydride copolymer, a styrene-maleic anhydride ester copolymer, a vinylmethyl ether-maleic anhydride copolymer,an ethylene-maleic anhydride copolymer, a vinyl-butyl ether-maleic anhydride copolymer, an acrylonitrile-methyl acrylate-maleic anhydride copolymer, a buthyl acrylate-styrene-maleic anhydride copolymer and the like.

Examples of the enteric polyvinyl derivative include: polyvinylacetate phthalate, polyvinylbutylate phthalate, polyvinylalcohol acetate phthalate and the like.

Among the compounds, it is preferred that the enteric material is the enteric acrylic acid-based copolymer or the enteric cellulose derivative. This makes it possible to rapidly dissolve the intestinal collapse-controlling layer 12a by being in contact with the body fluid in the intestines, while reliably preventing the intestinal collapse-controlling layer 12a from being collapsed in the oral cavity or the stomach. In the case where the methacrylic acid-methyl methacrylate copolymer, hydroxy-propyl-methyl cellulose phthalate or hydroxy-propyl-methyl cellulose acetate succinate is used as the enteric material from the compounds described above, the above effects are exhibited notably.

Furthermore, a mass-average molecular weight of the enteric polymer is not particularly limited, but is preferably in the range of 5,000 to 500,000 and more preferably in the range of 10,000 to 300,000. This makes it possible to enhance the stability of the shape of the intestinal collapse-controlling layer 12a. Furthermore, it is possible for the intestinal collapse-controlling layer 12a to quickly be dissolved by being in contact with the body fluid in the intestines. In addition, it is possible to obtain excellent adhesion between the intestinal collapse-controlling layer 12a and adjacent layers thereof (the intestinal medicine-containing layer 11 and the gel-forming layer 13a).

Furthermore, an amount of the enteric material contained in the intestinal collapse-controlling layer 12a is preferably in the range of 60 to 99 mass% and more preferably in the range of 75 to 95 mass%. This makes it possible to rapidly dissolve the intestinal collapse-controlling layers 12a by being in contact with the body fluid in the intestines, while reliably preventing the intestinal collapse-controlling layer 12a from being collapsed in the oral cavity or the stomach.

Furthermore, it is preferred that the intestinal collapse-controlling layer 12a includes a plasticizer of a predetermined amount. This makes it possible to reliably prevent cracks of the intestinal collapse-controlling layer 12a and improve the adhesion between the intestinal collapse-controlling layer 12a and the intestinal medicine-containing layer 11, and improve the adhesion between the intestinal collapse-controlling layer 12a and the gel-forming layer 13a. When the orally-administered agent 1a is swallowed, it is possible to reliably prevent delaminating from occurring between the gel-forming layer 13a and the intestinal collapse-controlling layer 12a and between the intestinal medicine-containing layer 11 and the intestinal collapse-controlling layer 12a.

Examples of materials to be used as the plasticizer of the intestinal collapse-controlling layer 12a include, but not particularly limited to, propylene glycol, polyethylene glycol, polypropylene glycol, glycerin triacetate, diethyl phthalate, triethyl citrate, lauric acid and the like. These materials may be used singly or in combination of two or more of them.

In the case where the plasticizer is contained in the intestinal collapse-controlling layer 12a, a content thereof is preferably in the range of 1 to 40 mass% and more preferably in the range of 5 to 25 mass% with respect to the intestinal collapse-controlling layer 12a.

Furthermore, the intestinal collapse-controlling layer 12a may include a base (namely, a base agent for the intestinal collapse-controlling layer) except for the enteric material as described above.

Examples of the base used in the intestinal collapse-controlling layer 12a include, but are not particularly limited to, starch such as α-starch, oxidized starch, carboxymethyl starch sodium, hydroxypropyl starch, dextrin and dextran; derivatives of starch; saccharides such as saccharose, maltose, lactose, glucose, fructose, pullulan, xanthane gum, cyclodextrin, xylitol, mannitol and sorbitol; shellac; polyvinylacetal diethylamino acetate; polyvinyl acetate; polyvinyl alcohol; polyvinyl pyrrolidone; a vinylacetate-vinyl pyrrolidone copolymer; natural rubbers such as Arabic gum and tragacanth gum; polyglucosamines such as chitin and chitosan; proteins such as gelatin, casein and soybean protein; titanium oxide; calcium monohydrogen phosphate; calcium carbonate; talc; stearate; magnesium aluminometasilicate; magnesium silicate; and silicic anhydride. One or more of these bases may be used independently or in combination according to the purposes of including the base into the collapse-controlling layer 12a.

In the case where the base is contained in the intestinal collapse-controlling layer 12a, a content thereof is preferably in the range of 1 to 25 mass%.

Furthermore, the intestinal collapse-controlling layer 12a may include different materials from those contained in the intestinal medicine-containing layer 11. By using the different materials between the intestinal collapse-controlling layer 12a and the intestinal medicine-containing layer 11, the orally-administered agent 1a is capable of releasing the different materials in different parts or at a different timing.

Furthermore, the intestinal collapse-controlling layer 12a may include other components than the materials described above. For example, the intestinal collapse-controlling layer 12a may include: an antiseptic agent such as methyl hydroxybenzoate and propyl hydroxybenzoate; a coloring agent such as an edible lake pigment; a masking agent such as a sweetener; and the like. A content thereof may be 5 mass% or less with respect to the intestinal collapse-controlling layer 12a.

Furthermore, it is preferred that it is difficult for the intestinal collapse-controlling layer 12a to be collapsed in other body cavities than the intestines. Concretely, it is preferred that it is difficult for the intestinal collapse-controlling layer 12a to be collapsed in the oral cavity and the stomach. Therefore, it is possible to reliably prevent the medicine contained in the intestinal medicine-containing layer 11 from being released into other body cavities than the intestines by collapse of the collapse-controlling layer 12a. As a result, the orally-administered agent 1a can release the medicine reliably into the intestines.

More specifically, it is preferred that the intestinal collapse-controlling layer 12a is not collapsed within three minutes by an artificial saliva of 37°C, more preferred that it is not collapsed within five minutes and even more preferred that it is not collapsed within ten minutes. Such an artificial saliva is a liquid in which NaCl of 0.08 mass%, KCl of 0.12 mass%, MgCl₂ of 0.01 mass%, CaCl₂ of 0.01 mass%, K₂HPO₄ of 0.03 mass% and CMC-Na of 0.10 mass% are added to purified water.

More specifically, it is necessary that the intestinal collapse-controlling layer 12a is not collapsed within less than 120 minutes with respect to a first test liquid (pH: 1.2) of 37°C used in a collapse test defined in Japanese Pharmacopoeia.

Furthermore, even more specifically, it is preferred that the intestinal collapse-controlling layer 12a is collapsed within less than 20 minutes with respect to a second test liquid (pH: 6.8) of 37°C used in a collapse test defined in Japanese Pharmacopoeia, more preferably less than 10 minutes and even more preferably less than 3 minutes.

By the above property of the intestinal collapse-controlling layer 12a, it is possible to reliably prevent the intestinal collapse-controlling layer 12a from being collapsed by the gastric juice and the saliva within the stomach and the oral cavity, respectively. Accordingly, it is possible to reliably prevent the medicine contained in the intestinal medicine-containing layer 11 from being dissolved and released into the oral cavity and the stomach. Furthermore, the intestinal collapse-controlling layer 12a is collapsed reliably in the intestines, and the medicine contained in the intestinal medicine-containing layer 11 is released reliably into the intestines.

Furthermore, a thickness of the intestinal collapse-controlling layer 12a is not particularly limited, but can appropriately adjust that according to parts to release the medicine in the intestines. Such a thickness is preferably in the range of 1 to 200 µm and more preferably in the range of 5 to 100 µm. This makes it possible for the orally-administered agent 1a to reliably release the medicine into the intestines.

### <Gel-forming Layer>

The gel-forming layer 13a is provided on the upper surface of the intestinal collapse-controlling layer 12a. On the other hand, the gel-forming layer 13b is provided on the lower surface of the intestinal collapse-controlling layer 12b.

Hereinafter, only the gel-forming layer 13a will be representatively described below, because the gel-forming layers 13a and 13b have substantially the same configuration.

The gel-forming layer 13a is a layer that can be swelled and gelatinized by absorbing water. As described above, the gel-forming layer 13a absorbs water contained in the saliva rapidly within the oral cavity, thereby forming a gel. By doing so, softness is rapidly given to the orally-administered agent 1a in the oral cavity, so that it becomes possible to swallow the orally-administered agent 1a with ease.

The gel-forming layer 13a contains a gel-forming agent that can be swelled and gelatinized by absorbing water. The gel-forming layer 13a containing such a gel-forming agent can form a gel by easily and rapidly absorbing water existing around the gel-forming layer 13a. Furthermore, the gel-forming agent can adjust an amount and property (pH etc.) of water to be in contact with the intestinal collapse-controlling layer 12a, so that the intestinal collapse-controlling layer 12a can be reliably collapsed at the intended parts of the body cavity.

Examples of the gel-forming agent include, but are not particularly limited to: an anionic polymer; starch; derivatives of starch; agar; arabinogalactan; galactomannan; dextranprotein; and the like. These materials may be used singly or in combination of two or more of them.

Among these materials, it is preferred that the gel-forming agent includes the anionic polymer. The anionic polymer is a polymer having anionic groups or salts thereof. The anionic groups contain carboxyl groups, sulfonic groups, phosphate groups and the like. The anionic groups are preferably the carboxyl groups. These anionic groups may include a base neutralized by salifiable cations. To be concrete, examples of the anionic polymer include: a polymer containing the carboxyl groups such as polyacrylic acid, polymethacrylic acid, polyitaconic acid, a carboxy vinyl polymer, carboxymethyl cellulose, carboxymethylhydroxyethyl cellulose, alginic acid, heparin, hyaluronic acid, carrageenan, and pectinic acid; salts of these polymers; a polymer containing the sulfonic groups such as polystyrene sulfonate, polyethylene sulfonate, and polyvinyl sulfonate; salts of these polymers; and the like. In this regard, examples of the salifiable cations include a sodium cation, a potassium cation, a monoethanolamine cation, a diethanolamine cation, a triethanolamine cation, an ammonium cation and the like. One or more of these cations can be used independently or in combination.

The anionic polymer can rapidly absorb water and can form the gel in a rapid manner. In addition, the anionic polymer is a component relatively hard to dissolve after formation of the gel. Therefore, the gel-forming layer 13a is reliably kept in a gelatinized shape within the oral cavity even after the formation of the gel. Furthermore, in the case where the polymer containing the carboxyl groups in the anionic polymer is used, the effects as described above are exhibited more conspicuously.

In such a case, a viscosity at 20°C of an aqueous solution of 0.2 mass% of the anionic polymer is preferably in the range of 1500 to 50,000 mPa·s and more preferably in the range of 10,000 to 20,000 mPa·s. This enables the gel-forming layer 13a to rapidly absorb the water and to form the gel in the rapid manner. The gel-forming layer 13a is reliably kept in the gelatinized shape.

In the case where the anionic polymer is used as the gel-forming agent, it may be possible to cross-link the anion polymer through the use of a cross-linking agent. This ensures that the gelatinized gel-forming layer 13a is surely prevented from dissolution within the oral cavity.

The cross-linking can be performed by the cross-linking agent that varies with a kind of molecules to be cross-linked. In the case where the anionic polymer is used as the gel-forming agent, as the cross-linking agent for cross-linking the anionic polymer, e.g., a polyvalent metal compound can be used. The polyvalent metal compound cross-links the anionic polymer as follows: When the gel-forming agent contained in the gel-forming layer 13a, that is, the anionic polymer is swelled and gelatinized within the oral cavity of the patient by the water contained in saliva etc., the polyvalent metal compound is ionized to thereby generate a polyvalent metal ion. Then, the polyvalent metal ion cross-links the anionic polymer contained in the gel-forming layer 13a. Accordingly, even if the sufficiently cross-linked gel-forming agent is not contained in the gel-forming layer 13a preliminarily, a gel having great enough strength is formed in the gel-forming layer 13a. On the other hand, the polyvalent metal ion is easily eluted from the gel-forming layer 13a to the gastric juice within the stomach, thereby enabling the gel-forming layer 13a to be collapsed reliably. As a result, the intestinal collapse-controlling layer 12 is easily collapsed by reliably being in contact with the body fluid within the intestines following the stomach, so that the medicine contained in intestinal medicine-containing layer 11 is reliably released into the intestines.

Examples of the polyvalent metal compound include, but are not particularly limited to, calcium chloride, magnesium chloride, aluminum chloride, aluminum sulfate, aluminum potassium sulfate, ferric chloride alum, ammonium alum, ferric sulfate, aluminum hydroxide, aluminum silicate, aluminum phosphate, iron citrate, magnesium oxide, calcium oxide, zinc oxide and zinc sulfate. One or more of these compounds can be used independently or in combination. Use of a trivalent metal compound among these compounds makes it possible to increase a cross-linking degree of the anionic polymer and enhancing physical strength of the gel-forming layer 13a. In addition, it is possible to reliably prevent the anionic polymer from being dissolved within the oral cavity.

A content of the gel-forming agent in the gel-forming layer 13a is preferably in the range of 5 to 90 mass% and more preferably in the range of 15 to 70 mass%, although it can be suitably adjusted depending on a kind of gel-forming agent or other factors. This enables the gel-forming layer 13a to rapidly absorb water. Furthermore, the gel-forming agent is reliably prevented from being dissolved within the oral cavity after gelatinization of the gel-forming agent.

In the case where the cross-linking agent is contained in the gel-forming layer 13a, a content of the cross-linking agent in the gel-forming layer 13a is preferably in the range of 0.1 to 2.5 mass% and more preferably in the range of 0.5 to 1.2 mass%. This makes it possible to surely prevent dissolution of the gel-forming layer 13a in the oral cavity while easily keeping the gel-forming layer 13a in the gelatinized shape after the gel-forming agent is gelatinized. In addition, it is possible to reduce a viscosity of a coating solution used as a raw material of the gel-forming layer 13a in the below-mentioned process of producing the orally-administered agent 1a, which makes it possible to efficiently form the gel-forming layer 13a.

The gel-forming layer 13a may contain a base (namely, a gel-forming base agent) which is a component contributing to stabilization of the shape of the gel-forming layer 13a. In other words, the base imparts a suitable degree of flexibility to the gel-forming layer 13a before the gel-forming agent is swelled by water, thereby preventing the orally-administered agent 1a from being cracked or damaged by an external force or other causes. After the gel-forming layer 13a has absorbed the water, the base serves to reliably keep the gel-forming layer 13a in the gelatinized shape, and prevent the gel from quickly flowing from the gel-forming layer 13a to the oral cavity.

Examples of the base used in the gel-forming layer 13a include, but are not particularly limited to, polyvinyl alcohol, polyvinyl pyrrolidone, polyvinyl acetate, polyvinyl acetate phthalate, hydroxyalkyl cellulose (e.g., hydroxypropyl cellulose, hydroxypropylmethyl cellulose, hydroxymethyl cellulose or hydroxyethyl cellulose), alkyl cellulose (e.g., methyl cellulose or ethyl cellulose), (metha)acrylate and the like. One or more of these compounds can be used independently or in combination.

In the case where the base is contained in the gel-forming layer 13a, a content of the base in the gel-forming layer 13a is preferably in the range of 20 to 85 mass% and more preferably in the range of 30 to 80 mass%.

It is preferred that the base contained in the gel-forming layer 13a is water-soluble. If the base is water-soluble, it becomes easy for water to get into the gel-forming layer 13a. As a result, it enables the gel-forming layer 13a to be rapidly swelled within the oral cavity, thereby rapidly forming the gel.

Examples of the water-soluble base include: polyvinyl alcohol; polyvinyl pyrrolidone; hydroxyalkyl cellulose such as hydroxypropyl cellulose, hydroxypropylmethyl cellulose; alkyl cellulose such as methyl cellulose; and the like.

In particular, in the case where polyvinyl alcohol is included in the base contained in the gel-forming layer 13a, the polyvinyl alcohol can suppress the taste or odor of the medicine contained in the intestinal medicine-containing layer 11 from being released into the oral cavity. That is to say, the polyvinyl alcohol can also serve as a masking agent to be described later.

Furthermore, the gel-forming layer 13a may contain a water absorption promoter for promoting water absorption of the gel-forming layer 13a. If the gel-forming layer 13a contains the water absorption promoter, it becomes possible to sufficiently increase the water absorption speed of the gel-forming layer 13a within the oral cavity. Accordingly, it becomes possible to improve the swallowability of the orally-administered agent 1a.

As the water absorption promoter, it is possible to use, e.g., a component having relatively high water-solubility. This component having relatively high water-solubility is dissolved in water and therefore can transport water into the gel-forming layer 13a. Consequently, the gel-forming layer 13a can absorb water quickly.

When an aqueous solution of 5 mass% of the water absorption promoter is prepared, a viscosity at 37°C of the aqueous solution is preferably in the range of 0.3 to 5.0 mPa·s, more preferably in the range of 0.5 to 3.5 mPa·s and even more preferably in the range of 0.6 to 1.8 mPa·s. As an indicator of water solubility of the water absorption promoter, it is possible to use, e.g., the viscosity of the aqueous solution in which the water absorption promoter is dissolved. It is possible to think that the water solubility of the water absorption promoter is low as the viscosity of the aqueous solution grows low. That is, if the viscosity of the aqueous solution of 5 mass% of the water absorption promoter falls within the range noted above, the water absorption promoter has suitably a high degree of the water solubility within the oral cavity. This makes it possible to suitably increase the water absorption speed of the gel-forming layer 13a. Furthermore, this also makes it possible to surely prevent the water absorption promoter from being suddenly dissolved and dispersed into the saliva.

Examples of the water absorption promoter include, but are not particularly limited to: glycols such as propylene glycol, polyethylene glycol, polypropylene glycol, polyoxyl stearate, polyoxyethylene polyoxypropylene glycol, polyoxyethylene-cured castor oil and the like; glycerin; and saccharides such as erythritol, sorbitol, xylitol, mannitol, inositol, maltitol, lactitol, glucose, xylose, mannose, fructose, galactose, sucrose, fructose, saccharose and the like. One or more of these compounds can be used independently or in combination.

It is preferred that the water absorption promoter contains glycerin among the compounds listed above. Glycerin is a component that has increased capability to promote the water absorption of the gel-forming layer 13a and has a function of being capable of imparting softness to the gel-forming layer 13a. Therefore, the orally-administered agent 1a has a suitable degree of flexibility until it is administered to a patient, which means that the orally-administered agent 1a is hardly broken or damaged by an external force. Furthermore, since glycerin has the functions as described above, the gel-forming layer 13a maintains the shape thereof and becomes soft in the oral cavity after the orally-administered agent 1a has been administrated. Therefore, it becomes possible to swallow the orally-administered agent 1a more easily. Furthermore, glycerin is a component of be capable of suppressing uncomfortable feeling by the bitter taste or odor of the medicine contained in the medicine-containing layer 11 within the oral cavity due to the sweet taste of glycerin.

A content of glycerin in the water absorption promoter is preferably in the range of 35 to 95 mass% and more preferably in the range of 40 to 90 mass%. This can increase the water absorption speed of the gel-forming layer 13a. Furthermore, this ensures that the orally-administered agent 1a becomes soft and easy to swallow.

Furthermore, it is preferred that the water absorption promoter contains a solid-state compound in an atmosphere of 1 atm at 25°C. Although the water absorption promoter is a component having relatively high water solubility, the addition of the solid-state compound under such an atmosphere makes it possible to prevent the orally-administered agent 1a from absorbing moisture during storage thereof. Therefore, it is possible to surely prevent the orally-administered agent 1a from being degraded during the storage thereof and to prevent the gel-forming layer 13a from being inadvertently gelatinized. Particularly, in the case where glycerin is contained in the water absorption promoter, the solid-state compound can prevent glycerin from flowing out (bleeding) during the storage of the orally-administered agent 1a.

Examples of such a solid-state compound include the glycols stated above and the afore-mentioned saccharides excepting glycerin.

It the case where the water absorption promoter contains the saccharides, the following advantageous effects can be obtained. More specifically, the saccharides can serve as a masking agent as described later, because they taste sweet and have increased capability to promote the water absorption of the gel-forming layer 13a. Furthermore, the sweet taste of the saccharides felt within the oral cavity by a patient helps accelerate secretion of the saliva. As a result, the orally-administered agent 1a shows increased swallowability. Furthermore, the saccharides and glycerin are similar in a chemical structure thereof, and therefore they have an extremely high affinity with respect to each other. Accordingly, if the water absorption promoter contains the saccharides and glycerin, the saccharides are capable of reliably holding glycerin in the gel-forming layer 13a and surely preventing glycerin from flowing out (bleeding) from the orally-administered agent 1a when storing the orally-administered agent 1a.

In the case where the water absorption promoter contains glycols among the compounds described above, the following advantageous effects can be obtained. Since the glycols show a good affinity to water and have a chain-like structure in a chemical structure thereof, glycols are a component that can be easily intertwined to molecules of glycols in themselves or other molecules than the glycols contained in the gel-forming layer 13a. Therefore, the glycols are linked to other molecules in the gel-forming layer 13a, thus maintaining the shape of the gel-forming layer 13a. This ensures that the gel-forming layer 13a is gelatinized with great ease while maintaining the shape of the gel-forming layer 13a. As a result, the orally-administered agent 1a shows especially high swallowability.

A content of the water absorption promoter in the gel-forming layer 13a is preferably in the range of 1 to 20 mass% and more preferably in the range of 3 to 17 mass%. This makes it possible to greatly increase the water absorption speed of the gel-forming layer 13a, while keeping the gel-forming layer 13a in a desired gel shape within the oral cavity.

Furthermore, the gel-forming layer 13a may contain a plasticizer. By containing the plasticizer in the gel-forming layer 13a, a proper degree of the softness is imparted. Examples of the plasticizer include glycerin triacetate, diethyl phthalate, triethyl citrate and lauric acid, one or more of which can be used independently or in combination.

The gel-forming layer 13a may contain a masking agent capable of suppressing the uncomfortable feeling by the taste or odor of the medicine contained in the intestinal medicine-containing layer 11. By containing the masking agent into the gel-forming layer 13a, it is possible for the gel-forming layer 13a to enhance the effect of suppressing the uncomfortable feeling by the taste or odor of the medicine (what is called a masking effect). Examples of the masking agent include: acidic-taste imparting agents such as citric acid, tartaric acid, fumaric acid and the like; sweetening agents such as saccharin, glycyrrhizinic acid and the like; mouth fresheners such as menthol, mentha oil, peppermint, spearmint and the like; natural or synthetic perfumes; and the like. One or more among these compounds can be used independently or in combination. The afore-mentioned saccharides as the water absorption promoter described above have a sweet taste and can serve as the masking agent.

The gel-forming layer 13a may contain other components than mentioned above. For example, the gel-forming layer 13a may contain: antiseptic agents such as methyl hydroxybenzoate, propyl hydroxybenzoate and the like; and coloring agents such as edible lake pigment and the like.

A thickness of the gel-forming layer 13a is preferably in the range of 10 to 1000 µm and more preferably in the range of 15 to 500 µm, although it may be suitably adjusted within an orally-administrable range of the orally-administered agent 1a.

### <Antiadhesive Layer>

The antiadhesive layer 14a is laminated on an upper surface of the gel-forming layer 13a at request. Such an antiadhesive layer 14a is provided as a surface layer constituting one surface of the orally-administered agent 1a. On the other hand, the antiadhesive layer 14b is laminated on a lower surface of the gel-forming layer 13b at request. Such an antiadhesive layer 14b is also provided as a surface layer constituting the other surface of the orally-administered agent 1a.

Furthermore, the antiadhesive layers 14a and 14b are rapidly dissolved by water contained in saliva within the oral cavity and have a function of preventing the orally-administered agent 1a from adhering to the inside wall in the oral cavity. In other words, when the orally-administered agent 1a is taken in the oral cavity, surface parts of the antiadhesive layers 14a and 14b are quickly dissolved by the saliva, thereby rapidly forming liquid-state films between the antiadhesive layers 14a and 14b and the inside wall of the oral cavity, respectively. As a result, it becomes easy for the orally-administered agent 1a to slide with respect to the inside wall. Therefore, the orally-administered agent 1a is prevented from being in contact with the inside wall of the oral cavity, so that it becomes difficult to adhere to the inside wall of the oral cavity. Furthermore, even if parts of the orally-administered agent 1a adhere to the inside wall, it becomes easy for the orally-administered agent 1a to peel off from the inside wall of the oral cavity. This makes it possible to prevent uncomfortable feelings from being brought by allowing the orally-administered agent 1a to adhere to the inside wall of the oral cavity, so that it becomes easy to swallow the orally-administered agent 1a. Furthermore, it is possible to reliably transfer the medicine contained in the intestinal medicine-containing layer 11 to the intended parts of the living body.

Particularly, the orally-administered agent 1a has the gel-forming layers 13a and 13b. Therefore, the orally-administered agent 1a becomes soft in the oral cavity. This makes it possible for the orally-administered agent 1a to more easily peel off from the inside wall by deformation of the orally-administered agent 1a with weak power, even if the parts of the orally-administered agent 1a adhere to the inside wall of the oral cavity.

Hereinafter, since the antiadhesive layers 14a and 14b are substantially identical with each other in configurations thereof, a description will be made on the antiadhesive layer 14a as representative.

The antiadhesive layer 14a is rapidly dissolved by water contained in saliva etc. within the oral cavity, and is mainly constituted of an antiadhesive agent which is capable of forming a aqueous-solution-state film around the orally-administered agent 1a.

Furthermore, in the antiadhesive agent as described above, a viscosity at 37°C of an aqueous solution of 5 mass% of the antiadhesive agent is preferably 50 mPa·s or less, and more preferably 40 mPa·s or less. As an indicator of adhesive property of the antiadhesive layer 14a with respect to the inside wall of the oral cavity, it is possible to use the viscosity of the aqueous solution in which a component (antiadhesive agent) constituting the antiadhesive layer 14a is dissolved. That is, the component constituting the antiadhesive layer 14a is quickly dissolved in water in the oral cavity and therefore an aqueous-solution-state film having a low viscosity is formed around the orally-administered agent 1a with ease as the viscosity of the aqueous solution grows low.

In the antiadhesive layer 14a, the phrase "mainly constituted of the antiadhesive agent" means that a content of the antiadhesive agent contained in the antiadhesive layer 14a is 50 mass% or higher. A content of the antiadhesive agent in the antiadhesive layer 14a is preferably 50 mass% or higher, and more preferably 70 mass% or higher. This makes it possible to conspicuously obtain the effects as described above.

The antiadhesive agent is not particularly limited as long as the viscosity characteristics as described above, that is, the viscosity at 37°C of the aqueous solution of 5 mass% of the antiadhesive agent falls within the range as described above. Examples of the antiadhesive agent include; a water-soluble polymer material such as hydroxyalkyl cellulose, polyethylene glycol, polypropylene glycol, polyvinyl alcohol, polyoxyl stearate, polyoxyethylene polyoxypropylene glycol, polyoxyethylene-cured castor oil, gum arabic, gelatin and the like; saccharides such as erythritol, sorbitol, xylitol, mannitol, inositol, maltitol, lactitol, glucose, xylose, mannose, fructose, galactose, sucrose, fructose, saccharose and the like; propylene glycol; glycerin; and the like. One or more of these compounds can be used independently or in combination. Furthermore, examples of the hydroxyalkyl cellulose include hydroxypropyl cellulose, hydroxypropylmethyl cellulose, methyl cellulose and the like.

Among the antiadhesive agents described above, it is preferred that the antiadhesive layer 14a includes the water-soluble polymer material as the antiadhesive agent. The water-soluble polymer material can be reliably dissolved in water and has a molecular chain having the appropriate length in a chemical structure thereof. Therefore, the molecular chain of the water-soluble polymer material in itself can be intertwined. Furthermore, in the case where the antiadhesive layer 14a includes a plurality of kinds of water-soluble polymer materials having different solubilities, the molecular chains of the water-soluble polymer materials having the different solubilities can be appropriately intertwined to each other. Therefore, when the water-soluble polymer materials dissolve from the antiadhesive layer 14a, it is possible to reliably allow the water-soluble polymer materials to unevenly exist around the orally-administered agent 1a in a state of an aqueous solution. For this reason, the orally-administered agent 1a is reliably prevented from adhering to the inside wall of the oral cavity for a long period of time. Furthermore, the water-soluble polymer material as described above can also serve as a base of the antiadhesive layer 14a, which is possible to produce the orally-administered agent 1a with ease. In addition, it is possible for the orally-administered agent 1a to reliably exhibit superior durability when storing the produced orally-administered agent 1a. In particularly, among the water-soluble polymer materials, in the case where at least one of polyethylene glycol, polyvinyl alcohol and hydroxypropyl cellulose is used, it is possible to conspicuously exhibit the effects as described above.

A mass-average molecular weight of the water-soluble polymer material as described above is preferably in the range of 5000 to 150000 and more preferably in the range of 10000 to 100000. This makes it possible for the water-soluble polymer material to sufficiently improve solubility with respect to water. After the water-soluble polymer material is dissolved, it is possible to reliably allow the water-soluble polymer material to unevenly exist around the orally-administered agent 1a in a state of an aqueous solution. Therefore, the orally-administered agent 1a is reliably prevented from reliably adhering to the inside wall of the oral cavity for a longer period of time.

Further, among the antiadhesive agents described above, the saccharides may be included in the antiadhesive layer 14a as the antiadhesive agent. The saccharides are capable of serving as a masking agent to mask tastes or odor of the medicine. Furthermore, the saccharides are components for accelerating secretion of the saliva in the oral cavity, Since the saccharides have superior solubility with respect to water, the saccharides not only serve as the antiadhesive agent but also have functions of helping that the gel-forming layer 13a is in contact with water.

Furthermore, the antiadhesive layer 14a may contain any components other than the components described above. For example, the antiadhesive layer 14a may include a plasticizer, a masking agent, an antiseptic agent, a coloring agent and the like as described above.

Furthermore, a mass of the antiadhesive layer 14a per unit area thereof is preferably in the range of 3 to 20 g/m² and more preferably in the range of 5 to 18 g/m². This makes it possible to allow an aqueous solution containing a component which has been eluted from the antiadhesive layer 14a to unevenly exist around the orally-administered agent 1a for a long period of time, while providing efficiently a thin orally-administered agent 1a (the antiadhesive layer 14a).

The orally-administered agent 1a as described above can produce, for example, by forming the antiadhesive layer on a supporting substrate before a step of producing the gel-forming layer according to the first embodiment, thereafter, using the same method as that of producing the orally-administered agent 1a according to the first embodiment. The antiadhesive layer 14a can be formed as follows:

Prepared first is a coating solution (namely, a coating solution for the antiadhesive layer) containing constituent materials of the antiadhesive layer. The coating solution for the antiadhesive layer can be prepared by dispersing or dissolving the constituent materials of the antiadhesive layer 14a as described above in a liquid medium such as purified water, ethanol or the like.

Next, the coating solution for the antiadhesive layer is applied or sprayed on a supporting substrate and then dried. This produces an antiadhesive layer.

The orally-administered agent 1a having the antiadhesive layer can be produced according to, e.g., the following processes.

### (Antiadhesive Layer Production Step)

Prepared first is a coating solution (namely, a coating solution for the antiadhesive layer) containing constituent materials of the antiadhesive layer 14a.

The coating solution for the antiadhesive layer can be prepared by dispersing or dissolving the constituent materials of the antiadhesive layer 14a as described above in a liquid medium such as purified water, ethanol or the like.

Next, the coating solution for the antiadhesive layer is applied or sprayed on the supporting substrate and then dried. This produces an antiadhesive layer to become the antiadhesive layer 14a on the supporting substrate. In this regard, it is to be noted that an antiadhesive layer to become the antiadhesive layer 14b can be also formed in the same manner as the process of producing the antiadhesive layer to become the antiadhesive layer 14a.

As the supporting substrate, it is possible to use, e.g., a glass plate, a plastic film or a release sheet, but is not limited to them.

### (Gel-forming Layer production Step)

Prepared next is a coating solution (namely, a coating solution for the gel-forming layer) containing constituent materials of the gel-forming layer 13a.

The coating solution for the gel-forming layer can be prepared by dispersing or dissolving the constituent materials of the gel-forming layer 13a as described above in a liquid medium such as purified water, ethanol or the like.

Next, the coating solution for the gel-forming layer is applied or sprayed on the antiadhesive layer formed on the supporting substrate and then dried. This produces a gel-forming layer to become the gel-forming layer 13a on the antiadhesive layer. In this regard, it is to be noted that a gel-forming layer to become the gel-forming layer 13b can be also formed in the same manner as the process of producing the gel-forming layer to become the gel-forming layer 13a.

### (Intestinal Collapse-Controlling Layer production Step)

Prepared next is a coating solution (namely, a coating solution for the intestinal collapse-controlling layer) containing constituent materials of the intestinal collapse-controlling layer 12a.

The coating solution for the intestinal collapse-controlling layer can be prepared by dispersing or dissolving the constituent materials of the intestinal collapse-controlling layer 12a as described above in a liquid medium such as purified water, ethanol or the like.

Next, the coating solution for the intestinal collapse-controlling layer is applied or sprayed on the gel-forming layer formed on the antiadhesive layer on the supporting substrate and then dried. This produces an intestinal collapse-controlling layer to become the intestinal collapse-controlling layer 12a. In this regard, it is to be noted that an intestinal collapse-controlling layer to become the intestinal collapse-controlling layer 12b can be also formed in the same manner as the process of producing the intestinal collapse-controlling layer to become the intestinal collapse-controlling layer 12a.

### (Intermediate Body Production Step)

Prepared next is a coating solution (namely, a coating solution for the intestinal medicine-containing layer) containing constituent materials of the intestinal medicine-containing layer.

The coating solution for the medicine-containing layer can be prepared by dispersing or dissolving the constituent materials of the intestinal medicine-containing layer 11 as described above in a liquid medium such as purified water, ethanol or the like.

Next, the coating solution for the intestinal medicine-containing layer is applied or sprayed on the intestinal collapse-controlling layer and then dried. This produces a precursor of the intestinal medicine-containing layer (namely, a precursor for the intestinal medicine-containing layer) on the intestinal collapse-controlling layer. In other words, an intermediate body for the orally-administered agent (hereinafter, simply referred to as an intermediate body) consisting of the precursor for the intestinal medicine-containing layer, the intestinal collapse-controlling layer, the gel-forming layer and the antiadhesive layer is produced. In this regard, the coating solution for the intestinal medicine-containing layer is applied on only parts of the intestinal collapse-controlling layer on which the intestinal medicine-containing layer is provided. The coating solution for the intestinal medicine-containing layer is not applied on other parts of the intestinal collapse-controlling layer. Another intermediate body is produced by the same process as that described above.

### (Thermal Compression Bonding Step)

Next, the two intermediate bodies produced in the intermediate body production step are thermally fusion-bonded together under a pressure so that the precursors of the intestinal medicine-containing layers of the intermediate bodies can be bonded to each other. Thus, the precursors of the two intestinal medicine-containing layers are fusion-bonded to form a single intestinal medicine-containing layer 11. Furthermore, the intestinal medicine-containing layer 11 is covered with the intestinal collapse-controlling layers 12a and 12b. In view of the above, obtained is the orally-administered agent 1a constituted from the laminate body which consists of the two antiadhesive layers 14a and 14b, the two gel-forming layers 13a and 13b, the two intestinal collapse-controlling layers 12a and 12b and the intestinal medicine-containing layer 11. The laminated body may be used as the orally-administered agent 1a as it stands, or may be processed by a method of punching it into an arbitrary shape, such as a circular shape, an elliptical shape or a polygonal shape, to produce the orally-administered agent 1a.

For example, an orally-administered agent 1a may include no antiadhesive layer. In this case, the orally-administered agent 1a is produced by performing only the steps after the step of producing the gel-forming layer without performing the step of producing the antiadhesive layer as described above.

Furthermore, the orally-administered agent 1a may be produced by, e.g., repeating the tasks of applying and drying the coating solution for the antiadhesive layer, the coating solution for the gel-forming layer, the coating solution for the intestinal collapse-controlling layer and the coating solution for the medicine-containing layer as described above.

### <Second Embodiment>

Next, a description will be made on an orally-administered agent in accordance with a second embodiment of the present invention.

FIG. 2 is a section view showing an orally-administered agent in accordance with a second embodiment.

Hereinafter, the orally-administered agent in accordance with the second embodiment of the present invention will now be described with reference to FIG. 2. The following description will be centered on the points differing from the first embodiment, with the same items omitted from the description.

As shown in FIG. 2, the orally-administered agent 1b of the present embodiment differs from that of the first embodiment in that surfaces of gel-forming layers 13c and 13d defining outer surfaces of the orally-administered agent 1b have a plurality of convex portions 131 and the antiadhesive layers 14a and 14b are not provided.

Provision of the convex portions 131 on outer surfaces of the gel-forming layers 13c and 13d as outermost surfaces of the orally-administered agent 1b makes it possible to reduce a contact area between the inside wall of the oral cavity and the orally-administered agent 1b. As a result, the orally-administered agent 1b is reliably prevented from adhering to the inside wall within the oral cavity. Therefore, it becomes easy for the medicine to reach the intended parts of the living body. In addition, provision of the convex portions 131 on the outer surfaces of the gel-forming layers 13c and 13d makes it possible to greatly increase a speed at which the gel-forming layers 13c and 13d absorb water from the saliva within the oral cavity. In other words, a contact area between the saliva and each of the gel-forming layers 13c and 13d can be increased by forming the convex portions 131, which results in a sharp increase in the water absorption speed of the gel-forming layers 13c and 13d. Thanks to the features noted above, the orally-administered agent 1b exhibits especially high swallowability.

The pitch p between the convex portions 131 of each of the gel-forming layers 13c and 13d is not particularly limited, but may be preferably in the range of 100 to 1,000 µm and more preferably in the range of 250 to 750 µm. This surely prevents the orally-administered agent 1b from adhering to the inside wall of the oral cavity, while greatly increasing the water absorption speed.

The width w of each of the convex portions 131 of each of the gel-forming layers 13c and 13d is not particularly limited, but may be preferably in the range of 20 to 300 µm and more preferably in the range of 50 to 250 µm. This surely prevents the orally-administered agent 1b from adhering to the inside wall of the oral cavity, while greatly increasing the water absorption speed.

The height d of each of the convex portions 131 of each of the gel-forming layers 13c and 13d is not particularly limited, but may be preferably in the range of 10 to 5,000 µm and more preferably in the range of 20 to 1,000 µm. This surely prevents the orally-administered agent 1b from adhering to the inside wall of the oral cavity, while greatly increasing the water absorption speed.

The gel-forming layers 13c and 13d having the convex portions 131 set forth above can be produced by, e.g., forming, on a surface of a supporting substrate, concave portions having a pattern complementary to that of the convex portions 131 to be formed on the gel-forming layer, applying the coating solution containing the constituent materials of the gel-forming layers 13c and 13d on the supporting substrate and drying the coating solution, when the gel-forming layers 13c and 13d are produced. Alternatively, the orally-administered agent 1b having no convex portions 131 may be produced, thereafter, the gel-forming layers 13c and 13d having the convex portions may be produced by, e.g., pressing a supporting substrate which have concave portions of a pattern complementary to that of the convex portions to be formed, against the gel-forming layers.

### <Third Embodiment>

Next, a description will be made on an orally-administered agent in accordance with a third embodiment of the present invention.

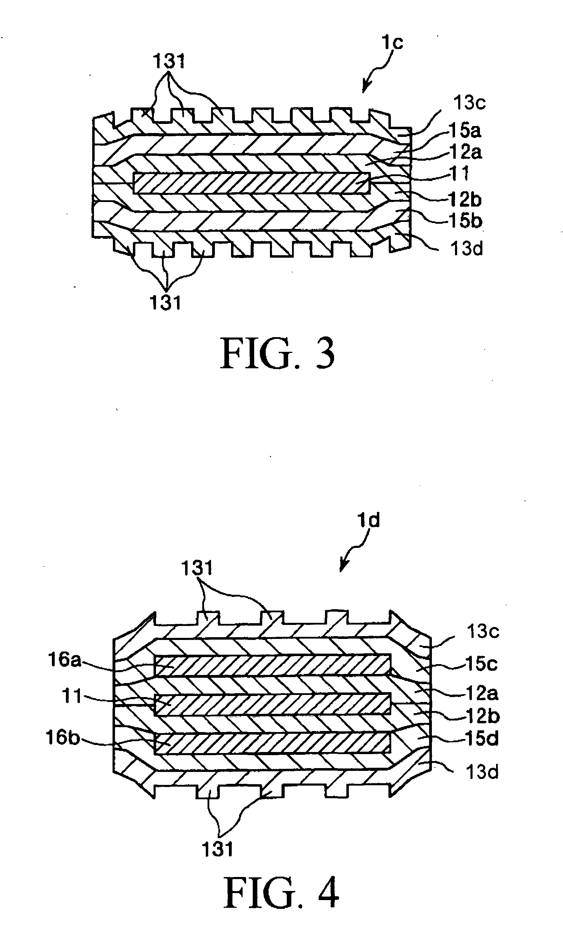

FIG. 3 is a section view showing an orally-administered agent in accordance with a third embodiment.

Hereinafter, the orally-administered agent in accordance with the third embodiment of the present invention will now be described with reference to FIG. 3. The following description will be centered on the points differing from the above embodiments, with the same items omitted from the description.

As shown in FIG. 3, the orally-administered agent 1c of the present embodiment differs from that of the second embodiment in that the orally-administered agent 1c has intragastric collapse-controlling layers 15a and 15b being capable of collapsing within the stomach.

The intragastric collapse-controlling layer 15a is provided between the intestinal collapse-controlling layer 12a and the gel-forming layer 13a. Furthermore, the intragastric collapse-controlling layer 15b is provided between the intestinal collapse-controlling layer 12b and the gel-forming layer 13b.

The intragastric collapse-controlling layers 15a and 15b are layers which are capable of collapsing by being in contact with the gastric juice. When such intragastric collapse-controlling layers 15a and 15b are provided on the upper surface and lower surface of the intestinal collapse-controlling layers 12a and 12b, respectively, the intestinal collapse-controlling layers 12a and 12b are prevented from being in contact with the body fluid such as the saliva while the orally-administered agent 1c is reached from the oral cavity to the stomach. On the other hand, the intragastric collapse-controlling layers 15a and 15b are collapsed in the stomach, so that the intestinal collapse-controlling layers 12a and 12b are exposed to the surfaces of the orally-administered agent 1c. Therefore, in the intestines following the stomach, the medicine contained in the intenstinal medicine-containing layer 11 is released by the collapse of the intestinal collapse-controlling layers 12a and 12b. As described above, by protecting the intestinal collapse-controlling layers 12a and 12b with the intragastric collapse-controlling layers 15a and 15b from the oral cavity to the intestines, the orally-administered agent 1c conveys the medicine reliably until the intestines. As a result, it becomes possible to more reliably release the medicine in the intestines.

Hereinafter, since the intragastric collapse-controlling layers 15a and 15b have the same configuration, a description will be made on the intragastric collapse-controlling layer 15a as a representative.

The intragastric collapse-controlling layer 15a include a stomach-soluble material which is dissolved by being in contact with the gastric juice. By including such a stomach-soluble material, the intragastric collapse-controlling layer 15a is in contact with the gastric juice within the stomach and then collapsed.

The stomach-soluble material, which can be used in the intragastric collapse-controlling layer 15a, is not particularly limited. For example, corresponds to a material being "practically insoluble" in water in the conditions defined in Japanese Pharmacopoeia Fifteenth Edition, and is a material of dissolving to an acid solution. More specifically, examples of the stomach-soluble material include: various kinds of inorganic compound such as calcium carbonate and sodium hydrogen carbonate; various kinds of stomach-soluble polymer; and the like. One or more of these compounds may be used independently or in combination.

Examples of the stomach-soluble polymer include: stomach-soluble polyvinyl derivatives such as polyvinyl aminoacetal and polyvinyl acetal diethylaminoacetate; stomach-soluble acrylic acid-based copolymer such as methylmethacrylate-butylmethacrylate-dimethylaminoethyl methacrylate copolymer, and aminoalkylmethacrylate copolymer E; and the like.

Among polymers described above, the stomach-soluble material is preferably the stomach-soluble acrylic acid-based copolymer and more preferably methylmethacrylate-butylmethacrylate-dimethylaminoethyl methacrylate copolymer. This makes it possible to enhance the stability of the shape of the intragastric collapse-controlling layer 15a. Furthermore, it is possible to quickly dissolve the stomach-soluble material by being in contact with the gastric juice within the stomach. In addition, it is possible to obtain excellent adhesion between the intragastric collapse-controlling layer 15a and adjacent layers thereof (the intestinal collapse-controlling layer 12a and the gel-forming layer 13a).

Furthermore, a mass-average molecular weight of the stomach-soluble polymer is not particularly limited, but is preferably in the range of 10,000 to 500,000 and more preferably in the range of 30,000 to 300,000. This makes it possible to enhance the stability of the shape of the intragastric collapse-controlling layer 15a. Furthermore, it is possible to quickly dissolve the stomach-soluble polymer by being in contact with the gastric juice within the stomach. In addition, it is possible to obtain excellent adhesion between the intragastric collapse-controlling layer 15a and adjacent layers thereof.

Furthermore, a content of the stomach-soluble material contained in the intragastric collapse-controlling layer 15a is preferably in the range of 60 to 100 mass% and more preferably in the range of 75 to 100 mass%. This makes it possible to reliably collapse the intragastric collapse-controlling layer 15a in the stomach, while exhibiting sufficiently excellent adhesion between the respective layers.

Furthermore, the intragastric collapse-controlling layer 15a may include a base (namely, a base agent for the intragastric collapse-controlling layer). Examples of the base used in the intragastric collapse-controlling layer 15a include, but are not particularly limited to, the same materials as those of the base of the intestinal collapse-controlling layer 12a as described above.

Furthermore, the intragastric collapse-controlling layer 15a may include a medicine. In this case, for example, the medicine can be released in the stomach by containing the medicine functioning at the stomach in the intragastric collapse-controlling layer 15a. Furthermore, in the intestines following the stomach, the medicine can be released from the intestinal medicine-containing layer 11. In other words, it is possible to release the different medicines at the parts of the living body, respectively, in one dosage of the orally-administered agent 1c.

Furthermore, the intragastric collapse-controlling layer 15a may include other components than the materials as described above.

Furthermore, it is preferred that it is difficult for the intragastric collapse-controlling layer 15a to be collapsed in other body cavities than the stomach. Concretely, it is preferred that it is difficult for the intragastric collapse-controlling layer 15a to be collapsed in the oral cavity.

More specifically, it is preferred that the intragastric collapse-controlling layer 15a is not collapsed within three minutes by an artificial saliva of 37°C, more preferred that it is not collapsed within five minutes and even more preferred that it is not collapsed within ten minutes. Such an artificial saliva is a liquid in which NaCl of 0.08 mass%, KCl of 0.12 mass%, MgCl₂ of 0.01 mass%, CaCl₂ of 0.01 mass%, K₂HPO₄ of 0.03 mass% and CMC-Na of 0.10 mass% are added to purified water.

More specifically, it is preferred that the intragastric collapse-controlling layer 15a is collapsed within less than twenty minutes with respect to a first test liquid (pH: 1.2) of 37°C used in a collapse test defined in Japanese Pharmacopoeia, more preferably less than ten minutes and even more preferably less than three minutes. Therefore, the intragastric collapse-controlling layer 15a can be collapsed by the gastric juice (hydrochloric acid aqueous) within the stomach. For these reasons, the medicine contained in the intestinal medicine-containing layer 11 is reliably dissolved and released into the intestines.

Furthermore, a thickness of the intragastric collapse-controlling layer 15a is not particularly limited, but is preferably in the range of 1 to 200 µm and more preferably in the range of 5 to 100 µm.

Furthermore, the orally-administered agent 1c, for example, is produced as the same manner as in the second embodiment described above. In other words, the orally-administered agent 1c can be produced by preparing a coating solution containing a constituent material of each of layers, applying the coating solution on a supporting substrate having concave portions in a predetermined order, drying that repeatedly to form intermediate bodies and further thermal-compressing the intermediate bodies.

### <Fourth Embodiment>

Next, a description will be made on an orally-administered agent in accordance with a fourth embodiment of the present invention.

FIG. 4 is a section view showing an orally-administered agent in accordance with a fourth embodiment.

Hereinafter, the orally-administered agent in accordance with the fourth embodiment of the present invention will now be described with reference to FIG. 4. The following description will be centered on the points differing from the above embodiments, with the same items omitted from the description.

As shown in FIG. 4, the orally-administered agent 1d of the present embodiment differs from that of the third embodiment in that the orally-administered agent 1d has intragastric medicine-containing layers 16a and 16b being capable of releasing the medicine within the stomach.

The intragastric medicine-containing layer 16a is provided on the upper surface of the intestinal collapse-controlling layer 12a and a lower surface of the intragastric collapse-controlling layer 15c. Furthermore, the intestinal collapse-controlling layer 12a and the intragastric collapse-controlling layer 15c are bonded to each other at parts in which they are not in contact with the intragastric medicine-containing layer 16a, thereby sealing the intragastric medicine-containing layer 16a. In other words, the intragastric medicine-containing layer 16a is provided on the upper surface of the intestinal collapse-controlling layer 12a so as to be covered by the intragastric collapse-controlling layer 15c.

The intragastric medicine-containing layer 16b is provided on the lower surface of the intestinal collapse-controlling layer 12b and an upper surface of the intragastric collapse-controlling layer 15d. Furthermore, the intestinal collapse-controlling layer 12b and the intragastric collapse-controlling layer 15d are bonded to each other at parts in which they are not in contact with the intragastric medicine-containing layer 16b, thereby sealing the intragastric medicine-containing layer 16b. In other words, the intragastric medicine-containing layers 16b is provided on the lower surface of the intestinal collapse-controlling layer 12b so as to be covered by the intragastric collapse-controlling layer 15d.

Due to the configuration as described above, the intragastric medicine-containing layers 16a and 16b can release the medicine by collapse of the intragastric collapse-controlling layers 15c and 15d in the stomach. Furthermore, in the intestines following the stomach, the medicine can be released from the intestinal medicine-containing layer 11. In other words, it is possible to release the different medicines at the parts of the living body, respectively, in one dosage of the orally-administered agent 1d.

Hereinafter, since the intragastric medicine-containing layers 16a and 16b have the same configuration, a description will be made on the the intragastric medicine-containing layer 16a as a representative.

The intragastric medicine-containing layer 16a is a layer which contains a medicine to be released in the stomach.

Examples of the medicine to be contained to the intragastric medicine-containing layer 16a include, but not limited thereto, the medicine to be used to the intestinal medicine-containing layer 11 as described above. It is preferred that used is a medicine of generating effects by being absorbed in the stomach.

A content of the medicine in the intragastric medicine-containing layer 16a is not particularly limited and may be suitably adjusted depending on a kind of medicine and the volume of the intragastric medicine-containing layer 16a. The content of the medicine is preferably in the range of 0.01 to 70 mass%, more preferably in the range of 0.01 to 40 mass% and even more preferably in the range of 0.01 to 35 mass%.

The intragastric medicine-containing layer 16a may include a base (namely, a base agent for the intragastric medicine-containing layer 16a). Examples of such a base include the same base as that for the intestinal medicine-containing layer as described above.

A thickness of the intragastric medicine-containing layer 16a is not particularly limited, but may be preferably in the range of 0.5 to 1000 µm, and more preferably in the range of 10 to 500 µm.

Furthermore, the orally-administered agent 1d, for example, is produced as the same manner in the second embodiment described above. In other words, the orally-administered agent 1d can be produced by preparing a coating solution containing a constituent material of each of layers, applying the coating solution on a supporting substrate having concave portions in a predetermined order, drying that repeatedly to form intermediate bodies and further thermal-compressing the intermediate bodies.

### <Fifth Embodiment>

Next, a description will be made on an orally-administered agent in accordance with a fifth embodiment of the present invention.

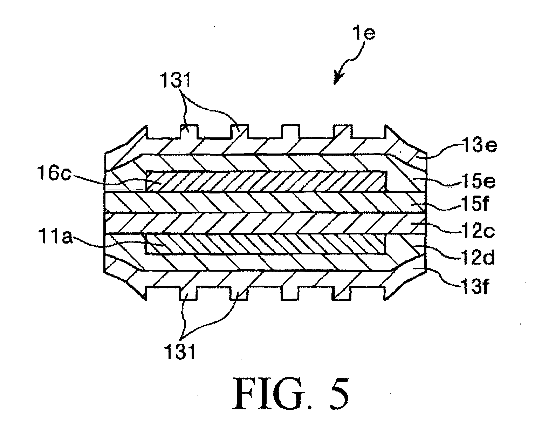

FIG. 5 is a section view showing an orally-administered agent in accordance with a fifth embodiment.

Hereinafter, the orally-administered agent in accordance with the fifth embodiment of the present invention will now be described with reference to FIG. 5. The following description will be centered on the points differing from the above embodiments, with the same items omitted from the description.

As shown in FIG. 5, an intestinal medicine-containing layer 11a is sandwiched and sealed by intestinal collapse-controlling layers 12c and 12d. Furthermore, an intragastric medicine-containing layer 16c is sandwiched and sealed by intragastric collapse-controlling layers 15e and 15f.

Furthermore, an upper surface of the intestinal collapse-controlling layer 12c and a lower surface of the intragastric collapse-controlling layer 15f are bonded to each other.

The gel-forming layer 13e is provided on an upper surface of the intragastric collapse-controlling layer 15e, which forms an outermost surface of an orally-administered agent 1e. Furthermore, the gel-forming layer 13f is provided on a lower surface of the intestinal collapse-controlling layer 12d, which forms an outermost surface of an orally-administered agent 1e.

Due to the configuration as described above, the orally-administered agent 1e can release the intended medicines at the intended parts of the living body, respectively. That is, the medicine contained in the intragastric medicine-containing layer 16c is released by the collapse of the intragastric collapse-controlling layers 15e and 15f in the stomach. In addition, the medicine contained in intestinal medicine-containing layer 11a is released by the collapse of the intestinal collapse-controlling layers 12c and 12d in the intestines.

Furthermore, the orally-administered agent 1e, for example, is produced as the same manner in the second embodiment described above. In other words, the orally-administered agent 1e can be produced by preparing a coating solution containing a constituent material of each of layers, applying the coating solution on a supporting substrate having concave portions in a predetermined order, drying that repeatedly to form intermediate bodies and further thermal-compressing the intermediate bodies.

While the illustrated embodiments of the present invention have been described hereinabove, the present invention shall not be limited thereto.

For example, an orally-administered agent according to the present invention may include additional arbitrary layers formed between respective layers. Furthermore, for example, the medicine-containing layer may be formed in a powder, compressive tablet or liquid manner.

Furthermore, for example, the medicine-containing layer may be exposed at a circumference portion of the orally-administered agent. Even such a configuration, the medicine is prevented from flowing involuntary since most of the medicine-containing layer is covered by the intestinal collapse-controlling layers which exist in the both surfaces of the medicine-containing layer.

Furthermore, for example, antiadhesive layers may be provided on outermost surfaces of the orally-administered agent. Convex portions may be formed on such antiadhesive layers.

### Examples

Next, concrete examples of the orally-administered agent according to the present invention will be described.

### 1. Production of Orally-Administered Agent

### (Example 1)

### (a) Antiadhesive layer Production Step

First, a coating solution A containing constituent materials of an antiadhesive layer was prepared.

Polyvinyl alcohol (Gohsenol EG05 produced by Nippon Synthetic Chemical Industry Co., Ltd.) as an antiadhesive agent was slowly added to purified water while stirring the same to obtain a mixture A. Thereafter, the mixture A was heated to 70°C and stirred for one hour to obtain the coating solution A.

Next, the coating solution A was sufficiently defoamed. Then, the coating solution A was flat-applied on an opposite surface of a release treatment surface of a polyethylene terephtalate film by using an applicator in which gaps between the release-treated polyethylene terephthalate film as a supporting substrate (SP-PET3811 produced by Lintec Corp.) and a blade of the applicator were adjusted so that an amount of the coating solution A after the applied coating solution A was dried became 15 g/m². Thereafter, the coating solution A thus applied was dried at 85°C for five minutes, thus producing the antiadhesive layer.

### (b) Gel-forming Layer Production Step

First, a coating solution B containing constituent materials of a gel-forming layer was prepared.

1.5 mass parts of calcium chloride (calcium chloride defined in Japanese Pharmacopoeia and produced by Tomita Pharmaceutical Co., Ltd.) was added to 1015 mass parts of purified water. The resultant mixture was stirred sufficiently to dissolve calcium chloride. As a result, an aqueous solution of calcium chloride was obtained. Then, 56.5 mass parts of polyacrylic acid (Carbopol 974P produced by CBC Co., Ltd., a viscosity of an aqueous solution of 0.2 mass% is 12100 mPa·s) was slowly added to the aqueous solution of calcium chloride while stirring the same to obtain a mixture B. After the addition of polyacrylic acid, the mixture B was stirred for about one hour. Next, 33.9 mass parts of polyvinyl alcohol (Gohsenol EG05 produced by Nippon Synthetic Chemical Industry Co., Ltd.) was slowly added to the mixture B while stirring the same to obtain a mixture C. After the addition of polyvinyl alcohol, the mixture C to which the respective materials had added was heated to 70 °C and stirred for about one hour. Next, 8.1 mass parts of glycerin (thick glycerin defined in Japanese Pharmacopoeia and produced by ADEKA Corp.) as a water absorption promoter was added to the mixture C and stirred for about ten minutes, thereby producing the coating solution B.

Next, the coating solution B was sufficiently defoamed. Then, the coating solution B was flat-applied on the antiadhesive layer produced in the step (a) by using an applicator in which gaps between the antiadhesive layer and a blade were adjusted so that an amount of the coating solution B after the applied coating solution B was dried became 50 g/m². Thereafter, the coating solution B thus applied was dried at 80°C for six minutes, thus producing the gel-forming layer.

### (c) Intestinal Collapse-controlling Layer Production Step

First, a coating solution C containing constituent materials of an intestinal collapse-controlling layer was prepared.

80 mass parts of methacrylic acid-methyl methacrylate copolymer (produced by Rheam Phama GmbH., a mass average molecular weight: 250,000) as an enteric material was added to 233 mass parts of ethanol, and then the same is sufficiently dispersed by using a homogenizer to obtain a mixture D. Thereafter, 20 mass parts of polyethyleneglycol (PEG1500 produced by Sanyo Chemical Industries, Ltd.) was slowly added to the mixture D while stirring the mixture D to obtain a mixture E. After the addition of polyethyleneglycol, the mixture E is stirred for about ten minutes to obtain the coating solution C.

Next, the coating solution C was sufficiently defoamed. Then, the coating solution C was flat-applied on the gel-forming layer produced in the step (b) by using an applicator in which gaps between the gel-forming layer and a blade were adjusted so that an amount of the coating solution C layer after the applied coating solution C was dried became 50 g/m². Thereafter, the coating solution C thus applied was dried at 90°C for five minutes, thus producing the intestinal collapse-controlling layer.

### (d) Intermediate Body Production Step

First, a coating solution D containing constituent materials of an intestinal medicine-containing layer was prepared.

2.5 mass parts of a blue dye (Blue No.2; dummy medicine) and 0.6 mass parts of titanium oxide (TIPAQUE CR-50 produced by Ishihara Sangyo Kaisha, Ltd.) were added to 53.7 mass parts of purified water and sufficiently dispersed through the use of a homogenizer to obtain a dispersion liquid. Thereafter, 13.8 mass parts of polyvinyl pyrrolidone (PVP K-90 produced by ISP Japan Ltd.) was slowly added to the dispersion liquid while stirring the same to obtain a mixture F. After the addition of polyvinyl pyrrolidone, the mixture F was stirred for about thirty minutes. Next, 4.0 mass parts of glycerin (thick glycerin defined in Japanese Pharmacopoeia and produced by ADEKA Corp.) was added to the mixture F and stirred for about five minutes, thereby producing the coating solution D.

Next, the coating solution D was sufficiently defoamed. Then, the coating solution D was applied on the intestinal collapse-controlling layer produced in the step (c) by using a screen printing. In this regard, the coating solution D was applied on a plurality of parts on the intestinal collapse-controlling layer so that an amount of the coating solution D after the applied coating solution D was dried became 50 g/m² and a shape of the intestinal medicine-containing layer precursor became a circular shape of which diameter was 10 mm. Thereafter, the coating solution D thus applied was dried at 80°C for five minutes, thus producing an intestinal medicine-containing layer precursor. Consequently, obtained were a laminated body (intermediate body) consisting of the intestinal medicine-containing layer precursor, the intestinal collapse-controlling layer, the gel-forming layer, and the antiadhesive layer. Another intermediate body was formed in the same process as those in the above steps (a) to (d).

### (e) Thermal Compression Bonding Step

The two intermediate bodies produced in the step (d) were thermally fusion-bonded together at a temperature of 100°C, under the conditions of a pressure of 1 kgf/cm² and for one second so that the intestinal medicine-containing layer precursors were bonded to each other. Next, the polyethylene terephthalate film was peeled off from each anthiadhesive layer, thereby producing a laminated body in which the antiadhesive layer, the gel-forming layer, the intestinal collapse-controlling layer, the intestinal medicine-containing layer, the intestinal collapse-controlling layer, the gel-forming layer, and the antiadhesive layer are laminated in this order. The laminated body was punched so that a circular film having a diameter of 15 mm at a point centered a central portion of the intestinal medicine-containing layer was obtained. Consequently, the orally-administered agent as shown in FIG. 1 was obtained. Furthermore, in the orally-administered agent, two intestinal collapse-controlling layers were bonded to each other, so that the intestinal medicine-containing layer was sandwiched and sealed (covered) by the two intestinal collapse-controlling layers.

### (Examples 2 to 6)

In each of the Examples 2 to 6, an orally-administered agent was obtained in the same manner as in the Example 1, except that the kind and the content of each of the constituent materials of the gel-forming layer, the intestinal collapse-controlling layer, and the antiadhesive layer were changed as shown in Tables 1 and 2.

### (Example 7)

An orally-administered agent as shown in FIG. 2 was obtained in the same manner as in the Example 1, except that (a) the antiadhesive layer production step was not performed and the application conditions of the coating solution B (production conditions of the gel-forming layer) were changed as mentioned below.

The coating solution B was sufficiently defoamed. Next, the coating solution B was flat-applied on a polyethylene terephthalate film by using an applicator in which gaps between the polyethylene terephthalate film and a blade were adjusted so that an amount of the coating solution B after the applied coating solution B was dried became 20 g/m². The polyethylene terephthalate film had concave portions (having the mouth size of 450x450 µm, the depth of 30 µm and the bottom size of 184x184 µm) provided in a grid pattern at a pitch of 550 µm. Thereafter, the coating solution B thus applied was dried at 80°C for five minutes, thus producing the gel-forming layer. The gel-forming layer thus produced was provided with convex portions having the height of about 30 µm, the width of about 450 µm and the pitch of about 550 µm, the shape of which was transferred from the concave portions of the polyethylene terephthalate film.

### (Examples 8 and 9)

In each of the Examples 8 and 9, an orally-administered agent was obtained in the same manner as in the Example 7, except that the kind and the content of each of the constituent materials of the gel-forming layer, and the intestinal collapse-controlling layer were changed as shown in Table 1.

### (Example 10)

An orally-administered agent as shown in FIG. 3 was obtained in the same manner as in the Example 7, except that after (b) the gel-forming layer production step and before (c) the intestinal collapse-controlling layer production step, an intragastric collapse-controlling layer production step was performed as mentioned below.

### (f) Intragastric Collapse-Controlling Layer Production Step

First, a coating solution E containing constituent materials of an intragastric collapse-controlling-layer was prepared.

80 mass parts of methyl methacrylate-butyl methacrylate-dimethylaminoethyl methacrylate copolymer (produced by Rheam Phama GmbH., a molecular weight: 150,000) as a stomach-soluble material was added to 233 mass parts of ethanol and then the same was stirred to dissolve that, so that a mixture G was obtained. Then, 20 mass parts of triethyl citrate was added to the mixture G. After the addition of triethyl citrate, the mixture G was stirred for about ten minutes to obtain the coating solution E.

Next, the coating solution E was sufficiently defoamed. Then, the coating solution E was flat-applied on the gel-forming layer produced in the step (b) by using an applicator in which gaps between the gel-forming layer and a blade were adjusted so that an amount of the coating solution E after the applied coating solution E was dried became 50 g/m². Thereafter, the coating solution E thus applied was dried at 90°C for five minutes, thus producing the intragastric collapse-controlling layer.

### (Example 11)

An orally-administered agent as shown in FIG. 4 was obtained in the same manner as in the Example 10, except that after (f) the intragastric collapse-controlling layer production step and before (c) the intestinal collapse-controlling layer production step, (g) an intragastric medicine-containing layer production step was performed as mentioned below.

### (g) Intragastric Medicine-containing Layer Production Step

First, a coating solution F containing constituent materials of an intragastric medicine-containing layer was prepared.

2.5 mass parts of a red dye (Red No.2; dummy medicine) and 0.6 mass parts of titanium oxide (TIPAQUE CR-50 produced by Ishihara Sangyo Kaisha, Ltd.) were added to 53.7 mass parts of purified water and sufficiently dispersed through the use of a homogenizer to obtain a mixture H. Thereafter, 13.8 mass parts of polyvinyl pyrrolidone (PVP K-90 produced by ISP Japan Ltd.) was slowly added to the mixture H while stirring the same. After the addition of polyvinyl pyrrolidone, the mixture H was stirred for about thirty minutes. Next, 4.0 mass parts of glycerin (thick glycerin defined in Japanese Pharmacopoeia and produced by ADEKA Corp.) was added to the mixture H and stirred for about five minutes, thereby producing the coating solution F.

Next, the coating solution F was sufficiently defoamed. Then, the coating solution F was applied on the intragastric collapse-controlling layer produced in the step (f) by using a screen printing. In this regard, the coating solution F was applied on a plurality of parts on the intragastric collapse-controlling layer so that an amount of the coating solution F after the applied coating solution F was dried became 50 g/m² and a shape of an intragastric medicine-containing layer precursor became a circular shape of which diameter was 10 mm. Thereafter, the coating solution F thus applied was dried at 80°C for five minutes. In this regard, the coating solutions F and D were applied so that a shape and a position of the intragastric medicine-containing layer are the same as those of the intestinal medicine-containing layer.

### (Example 12)

First, coating solutions B to F were prepared in the same manner as in the Examples 7, 10 and 11.

Next, by using these coating solutions B to F, obtained were an intermediate body A, in which the gel-forming layer, the intestinal collapse-controlling layer, the intestinal medicine-containing layer, and the intestinal collapse-controlling layer were laminated in this order, and an intermediate body B, in which the gel-forming layer, the intragastric collapse-controlling layer, the intragastric medicine-containing layer, the intragastric collapse-controlling layer were laminated in this order. The respective layers in these intermediate bodies were formed by applying the coating solutions B to F corresponding to the respective layers on the polyethylene terephthalate film one after another and drying them. The polyethylene terephthalate film had concave portions (having the mouth size of 450x450 µm, the depth of 30 µm and the bottom size of 184x184 µm) provided in a grid pattern at a pitch of 550 µm. In this regard, the conditions of forming the respective layers were set in the same manner as in the Examples 7, 10 and 11.

Next, the intermediate body A was attached to the intermediate body B so as to bond the intestinal collapse-controlling layer and the intragastric collapse-controlling layer together. Then, they were thermally fusion-bonded together at a temperature of 100°C, under the conditions of a pressure of 1 kgf/cm² and for one second.

### (Comparative Example 1)

An orally-administered agent was obtained in the same manner as in the Example 1, except that the antiadhesive layers and the gel-forming layers were not provided.

### (Comparative Example 2)

An orally-administered agent was obtained in the same manner as in the Example 1, except that the gel-forming layers and the intestinal collapse-controlling layers were not provided.

Tables 1 and 2 shown the constituent materials of the intestinal collapse-controlling layer, the gel-forming layer and the antiadhesive layer of the orally-administered agents obtained in the respective Examples and the respective Comparative Examples and the contents thereof. In Tables 1 and 2, the name "MM" signifies methacrylic acid-methyl methacrylate copolymer (mass-average molecular weight: 250,000), the name "HPMCP" signifies hydroxypropylmethylcellulose phthalate (mass-average molecular weight: 45,000), the name "HPMCS" signifies hydroxypropylmethylcellulose acetate succinate (mass-average molecular weight: 20,000), the name "PEG" signifies polyethylene glycol (PEG1500 produced by Sanyo Chemical Industries, Ltd.), the name "PAA" signifies polyacrylic acid (Carbopol 974P produced by CBC Co., Ltd.), the name "EG05" signifies polyvinyl alcohol (Gohsenol EG05 produced by Nippon Synthetic Chemical Industry Co., Ltd.), the name "EG40" signifies polyvinyl alcohol (Gohsenol EG40 produced by Nippon Synthetic Chemical Industry Co., Ltd.), the name "G" signifies glycerin (thick glycerin defined in Japanese Pharmacopoeia and produced by ADEKA Corp.), the name "Man" signifies mannitol, the name "Xyl" signifies xylitol, the name "PEP" signifies polyoxyethylene (105) polyoxypropylene (5) glycol (PEP-101 having the HLB of 20 and the molecular weight of 4000 to 5500, which is produced by Freund Corporation), the name "HPC" signifies hydroxypropyl cellulose (HPC(L) having the mass-average molecular weight of 60000, which is produced by NIPPON SODA CO., LTD.). Furthermore, the viscosity of the water absorption promoter in the Table 1 and the viscosity of the antiadhesive agent in the Table 2 denote a viscosity at 37°C of an aqueous solution of 5 mass% of the water absorption promoter and the antiadhesive agent, respectively. Each viscosity was measured with an E-type viscometer (a product of Tokimec, Inc.). In this regard, in the Examples 1-3, 7, and 10-12 in Table 1, the viscosity of the water absorption promoter denotes a viscosity of an aqueous solution of 5 mass% of glycerin, and in other Examples in Table 1, the viscosity of the water absorption promoter denotes a viscosity of an aqueous solution of 5 mass% of other water absorption promoter than glycerin.

Table 1

**Table 1**

| | Intestinal collapse-controlling layer | | | | Gel-forming layer | | | | | | | | | | |
|---|---|---|---|---|---|---|---|---|---|---|---|---|---|---|---|
| | Enteric material | | Plasticizer | | Base | | Gel-forming agent | | Cross-linking agent | | Water absorption promoter | | | | |
| | Kind | Content [Mass parts] | Kind | Content [Mass parts] | Kind | Content [Mass parts] | Kind | Content [Mass parts] | Kind | Content [Mass parts] | Kind | Content [Mass parts] | Kind | Content [Mass parts] | Viscosity [mPa·s] |
| Ex.1 | **MM** | 80 | PEG | 20 | EG05 | 33.9 | PAA | 56.5 | CaCl₂ | 1.5 | G | 8.1 | - | - | 0.90 |
| Ex.2 | **HPMCS** | 80 | PEG | 20 | EG05 | 33.9 | PAA | 56. 5 | CaCl₂ | 1.5 | G | 8. 1 | - | - | 0.90 |
| Ex.3 | **HPMCP** | 80 | PEG | 20 | EG05 | 33.9 | PAA | 56.5 | CaCl₂ | 1.5 | G | 8.1 | - | - | 0.90 |
| Ex.4 | **MM** | 80 | PEG | 20 | EG40 | 66.8 | PAA | 22.2 | CaCl₂ | 0.6 | G | 7.9 | Man | 2.5 | 0.87 |
| Ex.5 | **MM** | 80 | PEG | 20 | EG05 | 66.8 | PAA | 22. 2 | CaCl₂ | 0.6 | G | 7.9 | Xyl | 2.5 | 1.08 |
| Ex.6 | **MM** | 80 | PEG | 20 | EG05 | 66.8 | PAA | 22.2 | CaCl₂ | 0.6 | G | 7.9 | PEP | 2.5 | 1. 62 |
| Ex.7 | **MM** | 80 | PEG | 20 | EG05 | 33.9 | PAA | 56.5 | CaCl₂ | 1.5 | G | 8.1 | - | - | 0.90 |
| Ex.8 | **HPMCS** | 80 | PEG | 20 | EG40 | 66.8 | PAA | 22.2 | CaCl₂ | 0.6 | G | 7.9 | Man | 2.5 | 0.87 |
| Ex.9 | **HPMCP** | 80 | PEG | 20 | EG05 | 66.8 | PAA | 22.2 | CaCl₂ | 0.6 | G | 7.9 | PEP | 2.5 | 1.62 |
| Ex.10 | **MM** | 80 | PEG | 20 | EG05 | 33.9 | PAA | 56.5 | CaCl₂ | 1.5 | G | 8.1 | - | - | 0.90 |
| Ex.11 | **MM** | 80 | PEG | 20 | EG05 | 33.9 | PAA | 56.5 | CaCl₂ | 1.5 | G | 8. 1 | - | - | 0.90 |
| Ex.12 | **MM** | 80 | PEG | 20 | EG05 | 33.9 | PAA | 56.5 | CaCl₂ | 1.5 | G | 8. 1 | - | - | 0.90 |
| Comp.Ex.1 | **MM** | 80 | PEG | 20 | - | - | - | - | - | - | - | - | - | - | - |
| Comp.Ex.2 | - | - | - | - | - | - | - | - | - | - | - | - | - | - | - |

Table 2

**Table 2**

| | Antiadhesive layer | | | | | |
|---|---|---|---|---|---|---|
| | Antiadhesive agent | | | | | |
| | Kind | Content [Mass parts] | Viscosity [mPa·s] | Kind | Content [Mass parts] | Viscosity [mPa·s] |
| Ex.1 | EG05 | 100 | 5.4 | - | - | - |
| Ex.2 | HPC | 100 | 27.6 | - | - | - |
| Ex.3 | EG05 | 75 | 5.4 | Man | 25 | 0.87 |
| Ex.4 | EG05 | 100 | 5.4 | - | - | - |
| Ex.5 | HPC | 100 | 27.6 | - | - | - |
| Ex.6 | EG05 | 75 | 5.4 | Xyl | 25 | 0.87 |
| Comp.Ex.2 | EG05 | 100 | 5.4 | - | - | - |

### 2. Evaluation of Collapse Property of Intestinal Collapse-controlling Layer

In the orally-administered agent of each of the Examples and the Comparative Examples, collapse property of the intestinal collapse-controlling layers was examined according to a collapse test defined in Japanese Pharmacopoeia Fifteenth Edition. The results were evaluated.

First, the coating solution B to form the intestinal collapse-controlling layer, which was used in the respective Examples and the respective Comparative Examples, was flat-applied on an opposite surface of a release treatment surface of a polyethylene terephthalate film (SP-PET3811 produced by Lintec Corp.) by using an applicator in which gaps between the polyethylene terephthalate film and a blade were adjusted so that an amount of the coating solution B after the applied coating solution B was dried became 50 g/m². Thereafter, the coating solution B thus applied was dried at 90°C for five minutes, thereby removing the polyethylene terephthalate film to obtain a film consisting of the intestinal collapse-controlling layer. The film was punched so that a shape thereof became circular shape having a diameter of 15 mm. By these processes, a sample to evaluate the collapse property was obtained.

An artificial saliva, a first liquid (pH: 1.2) and a second liquid (pH: 6.8) for the collapse test were used as a test liquid used in the collapse test. Such an artificial saliva is a liquid in which NaCl of 0.08 mass%, KCl of 0.12 mass%, MgCl₂ of 0.01 mass%, CaCl₂ of 0.01 mass%, K₂HPO₄ of 0.03 mass% and CMC-Na of 0.10 mass% are added to purified water. The collapse test was performed in the same manner as a method of handling a capsule material. In the respective test liquids, time until the sample was absolutely collapsed was measured. In this regard, it is to be noted that the test was performed three times to the respective test liquids to obtain an average value of the time values which were obtained by the tests of the three times. The average value was evaluated according to four criteria described below.

### [Evaluation Criteria According to Collapse Property Using Artificial Saliva]

A...Time until the intestinal collapse-controlling layer was collapsed was 10 minutes or more.
B...Time until the intestinal collapse-controlling layer was collapsed was 5 minutes or more but lower than 10 minutes.
C...Time until the intestinal collapse-controlling layer was collapsed was 3 minutes or more but lower than 5 minutes.
D...Time until the intestinal collapse-controlling layer was collapsed was lower than 3 minutes.

### [Evaluation Criteria According to Collapse Property Using First Liquid for Collapse Test]

A···Time until the intestinal collapse-controlling layer was collapsed was 120 minutes or more.
D···Time until the intestinal collapse-controlling layer was collapsed was lower than 120 minutes.

### [Evaluation Criteria According to Collapse Property Using Second Liquid for Collapse Test]

A···Time until the intestinal collapse-controlling layer was collapsed was lower than 3 minutes.
B···Time until the intestinal collapse-controlling layer was collapsed was 3 minutes or more but lower than 5 minutes.
C···Time until the intestinal collapse-controlling layer was collapsed was 5 minutes or more but lower than 20 minutes.
D···Time until the intestinal collapse-controlling layer was collapsed was 20 minutes or more.

### 3. Evaluation of Elution Property of Medicine

In the orally-administered agent of each of the Examples and the Comparative Examples, elution property of the medicine contained in the intestinal medicine-containing layer was tested according to an elution test (Rotating Basket Method) defined in Japanese Pharmacopoeia Fifteenth Edition. The results were evaluated.

The test was performed by using the artificial saliva (described above), a first liquid (pH: 1.2) and a second liquid (pH: 6.8) for the elution test as a test liquid. In each test liquid, time until the medicine was eluted from the intestinal medicine-containing layer to each test liquid (until the blue dye of the dummy medicine contained in the intestinal medicine-containing layer was eluted to each test liquid) was measured. In this regard, it is to be noted that the test was performed three times to the respective test liquids to obtain an average value of the time values which were obtained by the tests of the three times. The average value was evaluated according to four criteria described below. The measurement of the time until the medicine was started to elute from the intestinal medicine-containing layer to each test liquid was performed by visually confirming that the color of each of the test liquids was changed to the blue color. Furthermore, in the evaluation using the second liquid for the elution test in the Examples 10 and 11, the evaluation was performed by dipping the orally-administered agent of the Examples 10 and 11 into the artificial saliva for 10 minutes, then dipping that to the first liquid for the elution test for 120 minutes and thereafter dipping that to the second liquid for the elution test.

### [Evaluation Criteria According to Elution Property Using Artificial Saliva]

A···Time until the medicine was eluted from the intestinal medicine-containing layer to the artificial saliva was 10 minutes or more.
B···Time until the medicine was eluted from the intestinal medicine-containing layer to the artificial saliva was 5 minutes or more but lower than 10 minutes.
C···Time until the medicine was eluted from the intestinal medicine-containing layer to the artificial saliva was 3 minutes or more but lower than 5 minutes.
D···Time until the medicine was eluted from the intestinal medicine-containing layer to the artificial saliva was lower than 3 minutes.

[Evaluation Criteria According to Elution Property Using First Liquid for Elution Test]
A···Time until the medicine was eluted from the intestinal medicine-containing layer to the first liquid for the elution test was 120 minutes or more.
D···Time until the medicine was eluted from the intestinal medicine-containing layer to the first liquid for the elution test was lower than 120 minutes.

[Evaluation Criteria According to Elution Property Using Second Liquid for Elution Test]
A···Time until the medicine was eluted from the intestinal medicine-containing layer to the second liquid for the elution test was lower than 3 minutes.
B···Time until the medicine was eluted from the intestinal medicine-containing layer to the second liquid for the elution test was 3 minutes or more but lower than 5 minutes.
C···Time until the medicine was eluted from the intestinal medicine-containing layer to the second liquid for the elution test was 5 minutes or more but lower than 20 minutes.
D···Time until the medicine was eluted from the intestinal medicine-containing layer to the second liquid for the elution test was 20 minutes or more.

### 4. Evaluation of Adhesive Property

Gargling was conducted to cleanse the interior of the oral cavity. After two minutes, the orally-administered agent produced in each of the Examples and the Comparative Examples was put in the mouth without water so as to on purpose adhere to the palate with ease. Thereafter, it was confirmed whether or not each orally-administered agent adhered to the palate. In the case where each orally-administered agent adhered to the palate, it was confirmed whether or not the orally-administered agent could be peeled off from the palate by tongue. The results were evaluated according to the below five criteria to obtain evaluation values. In this regard, it is to be noted that the evaluation of the adhesive property was carried out five times to obtain five values. Then, an average value was obtained from the obtained five values as an overall evaluation.
1······A whole of one surface of the orally-administered agent adhered to the palate, and the adhered whole of the one surface could not be peeled off by tongue with ease.
2······A part of one surface of the orally-administered agent adhered to the palate, and the adhered part of the one surface could not be peeled off by tongue with ease.
3······Although a part or whole of one surface of the orally-administered agent adhered to the palate, the adhered part or whole of the one surface could be peeled off by tongue with ease.
4······Although a part or whole of one surface of the orally-administered agent adhered to the palate, the adhered part or whole of the one surface could be quickly peeled off.
5······The orally-administered agent hardly adhered to the palate.

### 5. Evaluation of Swallowability (Evaluation of Administrability)

Gargling was conducted to cleanse the interior of the oral cavity. After two minutes, each of the orally-administered agents produced in the respective Examples and the Comparative Examples was put into the oral cavity without water and swallowed. The swallowability of the orally-administered agent was evaluated according to the below five criteria. In this regard, it is to be noted that the evaluation was carried out five times to obtain five values. Then, an average value was obtained from the obtained five values as an overall evaluation. At the same time, it was confirmed and evaluated whether or not the orally-administered agent stuck in the throat, the airway and the esophagus when swallowing the orally-administered agent (safety of administrating).

### [Evaluation Criteria According to Swallowability]

1···The orally-administered agent was not swelled and gelatinized and could not be administrated without water.
2···The orally-administered agent was slightly swelled and gelatinized but could not be administrated without water.
3···The orally-administered agent was swelled and gelatinized but wanted to be administrated with water if possible.
4···The orally-administered agent was slowly swelled and gelatinized but could be administrated without water.
5···The orally-administered agent was quickly swelled and gelatinized but could be administrated without water.

These results are shown in Table 3.

Table 3

**Table 3**

| | Evaluation of collapse property of intestinal collapse-controlling layer | | | Evaluation of elution property of medicine | | | Evaluation of adhesive property | Evaluation of swallowability | |
|---|---|---|---|---|---|---|---|---|---|
| | Artificial Saliva | First liquid for test liquid | Second liquid for test liquid | Artificial Saliva | First liquid for test liquid | Second liquid for test liquid | | Swallowability | Sticking in the throat etc. |
| Ex.1 | A | A | A | A | A | A | 5.0 | 4.0 | No |
| Ex.2 | A | A | A | A | A | A | 4.6 | 4.0 | No |
| Ex.3 | A | A | A | A | A | A | 5.0 | 4.0 | No |
| Ex.4 | A | A | A | A | A | A | 5.0 | 5.0 | No |
| Ex.5 | A | A | A | A | A | A | 4.8 | 5.0 | No |
| Ex.6 | A | A | A | A | A | A | 5.0 | 5.0 | No |
| Ex.7 | A | A | A | A | A | A | 5.0 | 4.2 | No |
| Ex.8 | A | A | A | A | A | A | 5.0 | 5.0 | No |
| Ex.9 | A | A | A | A | A | A | 5.0 | 5.0 | No |
| Ex.10 | A | A | A | A | A | A | 5.0 | 4.0 | No |
| Ex.11 | A | A | A | A | A | A | 5.0 | 4.0 | No |
| Ex.12 | A | A | A | A | A | A | 5.0 | 4.0 | No |
| Comp.Ex.1 | A | A | A | A | A | A | 5.0 | 1.0 | Yes |
| Comp.Ex.2 | - | - | - | D | D | D | 5.0 | Dissolved | No |

With regard to the orally-administered agent of the Examples 11 and 12 having both the intestinal medicine-controlling layer and the intragastric medicine-containing layer, when the elution property of the medicine was evaluated by using the artificial saliva and the first liquid for the elution test, time until the medicine (Red dye of the dummy medicine) contained in the intragastric medicine-containing layer was eluted to the artificial saliva and the first liquid for elution test was measured. As a result, the time until the medicine contained in the intragastric medicine-containing layer was eluted to the artificial saliva was 10 minutes or more in the orally-administered agents of the Examples 11 and 12. Furthermore, the time until the medicine contained in the intragastric medicine-containing layer was eluted to the first liquid for the elution test was lower than 3 minutes in the orally-administered agents of the Examples 11 and 12.

As shown in Table 3, it was difficult for the orally-administered agent obtained in each of the Examples 1 to 12 to release the medicine (Blue dye of the dummy medicine) contained in the intestinal medicine-containing layer in the artificial saliva and the first liquid for the test liquid. However, the orally-administered agent obtained in each of the Examples 1 to 12 could reliably release the medicine contained in the intestinal medicine-containing layer in the second liquid for the test liquid. From the results, it was considered that even if the orally-administered agent obtained in each of the Examples 1 to 12 was in contact with the saliva in the oral cavity and the gastric acid in the stomach, the medicine contained in the intestinal medicine-controlling layer could be not released but could be released in the intestines reliably. Furthermore, as described above, in the orally-administered agent having both the intestinal medicine-controlling layer and the intragastric medicine-containing layer of the Examples 11 and 12, the medicine (red dye of the dummy medicine) contained in the intragastric medicine-controlling layer was released reliably into the first liquid for test liquid and the medicine (blue dye of the dummy medicine) contained in the intestinal medicine-controlling layer was suppressed from releasing. For these reasons, it was considered that each of the orally-administered agents of the Examples 11 and 12 could release the intended medicines in the stomach and the intestines, respectively.

Furthermore, the orally-administered agent obtained in each of the Examples exhibited superior swallowability and could be swallowed without water. Furthermore, it was difficult for the orally-administered agents having the antiadhesive layers (the Examples 1 to 6) and the orally-administered agents having the gel-forming layers of which surfaces had convex portions (the Examples 7 to 12) to adhere to the inside of the oral cavity. As a result, it had found that it was difficult for the orally-administered agent to adhere to the inside of the body cavity and it was easy to swallow the orally-administered agent. Accordingly, it was presumed that the orally-administered agent could be reliably delivered to the intended parts of the living body without adhering to the body cavity.

In contrast, in the orally-administered agent obtained in each of the Comparative Examples, no satisfactory results were obtained. In other words, the orally-administered agent of the Comparative Example 1 could not be administrated without water and stuck in the throat and the like with ease. Furthermore, the orally-administered agent of the Comparative Example 2 released the medicine in the artificial saliva and an acid solution (first liquid for test liquid) with ease. Accordingly, the orally-administered agent of the Comparative Example 2 also released the medicine within the oral cavity and the stomach with ease.

Furthermore, all of the water absorption promoter used in each of the Examples were solid-state compounds under the conditions of 1 atm at 25°C except for glycerin.

### EXPLANATION OF REFERENCE NUMERAL

1a, 1b, 1c, 1d and 1e: orally-administered agent
11 and 11a: intestinal medicine-containing layer
12a, 12b, 12c and 12d: intestinal collapse-controlling layer
13a, 13b 13c, 13d, 13e and 13f: gel-forming layer
131: convex portions
14a and 14b: antiadhesive layer
15a, 15b, 15c, 15d, 15e and 15f: intragastric collapse-controlling layer
16a, 16b and 16c: intragastric medicine-containing layer

### INDUSTRIAL APPLICABILITY

An orally-administered agent according to the present invention can be swallowed with ease and release a medicine in the intend parts (in particular, intestines) of the living body. Therefore, the orally-administered agent according to the present invention can be reliably used to, in particular, aged persons or infants. Accordingly, the orally-administered agent according to the present invention contributes to the development of industries such as pharmaceutical preparations.

## Claims

1. An orally-administered agent (1b;1c;1d;1e) comprising:
a medicine-containing layer (11;11a) containing a medicine and having surfaces;
two intestinal collapse-controlling layers (12a;12b;12c;12d) including an enteric material which is dissolved by being in contact with the intestinal body fluid, wherein the intestinal collapse-controlling layers (12a;12b;12c;12d) are provided directly or through an arbitrary layer on the surfaces of the medicine-containing layer (11;11a), respectively, and each of the intestinal collapse-controlling layers (12a;12b;12c;12d) has a surface opposite to the medicine-containing layer (11;11a); and
two gel-forming layers (13c;13d;13e;13f) containing a gel-forming agent that is swelled and gelatinized by absorbing water, wherein the gel-forming layers (13c;13d;13e;13f) are provided directly or through an arbitrary layer on the sides of the surfaces of the intestinal collapse-controlling layers (12a;12b;12c;12d), respectively,
wherein each of the gel-forming layers (13c;13d;13e;13f) has a surface provided with a plurality of convex portions and a pitch p between the convex portions is in a range of 100 to 1,000 µm, and
wherein the surfaces of the gel-forming layers (13c;13d;13e;13f) define outer surfaces of the orally-administered agent (1b;1c;1d;1e).

2. The orally-administered agent (1b;1c;1d;1e) as claimed in claim 1, wherein the medicine-containing layer (11;11a) is completely covered with the intestinal collapse-controlling layers (12a;12b;12c;12d).

3. The orally-administered agent (1b;1c;1d;1e) as claimed in claim 1, wherein the enteric material contains an enteric polymer.

4. The orally-administered agent (1b;1c;1d;1e) as claimed in claim 3, wherein the enteric polymer is an enteric acrylic acid-based copolymer selected from the group consisting of a methacrylic acid-methyl methacrylate copolymer, methacrylic acid-ethyl acrylate, a styrene-acrylic acid copolymer, a methyl acrylate-acrylic acid copolymer, a methyl acrylate-methacrylic acid copolymer, a buthyl acrylate- styrene-acrylic acid copolymer, and a methyl acrylatemethacrylic acid-octyl acrylate copolymer, or a cellulose derivative selected from the group consisting of hydroxy-propyl-methyl cellulose acetate succinate, hydroxy-propyl-methyl cellulose phthalate, hydroxypropyl-methyl acetate maleate, hydroxy-methyl-ethyl cellulose phthalate, cellulose acetate phthalate, cellulose acetate succinate, cellulose acetate trimellitate, cellulose acetate maleate, cellulose benzoate phthalate, cellulose propionate phthalate, methyl cellulose phthalate, ethyl-hydroxy-ethyl cellulose phthalate, hydroxy-methyl cellulose ethylphthalate, and cellulose acetate phthalate.

5. The orally-administered agent (1b;1c;1d;1e) as claimed in claim 1, wherein the gel-forming layers contain an anionic polymer.

6. The orally-administered agent (1b;1c;1d;1e) as claimed in claim 1, wherein the gel-forming layers (13c;13d;13e;13f) contain a water absorption promoter for promoting water absorption of the gel-forming layers (13c;13d;13e;13f).

7. The orally-administered agent (1b;1c;1d;1e) as claimed in claim 6, wherein when an aqueous solution of 5 mass% of the water absorption promoter is prepared, a viscosity at 37°C of the aqueous solution measured with an E-type viscometer is in a range of 0.3 to 5.0 mPa·s.

8. The orally-administered agent (1b;1c;1d;1e) as claimed in claim 1, wherein the intestinal collapse-controlling layers (12a;12b;12c;12d) contain a medicine which is different from the medicine contained in the medicine-containing layer (11;11a).

9. The orally-administered agent (1 c) as claimed in claim 1 further comprising two intragastric collapse-controlling layers (15a;15b) containing a stomach-soluble material which is dissolved by being in contact with gastric juice, wherein each one of the two intragastric collapse-controlling layers (15a;15b) is respectively provided between one of the two intestinal collapse-controlling layers (12a;12b) and the corresponding gel-forming layer (13c;13d).

10. The orally-administered agent (1d) as claimed in claim 1 further comprising
two intragastric collapse-controlling layers (15c;15d) containing a stomach-soluble material which is dissolved by being in contact with gastric juice, wherein each one of the two intragastric collapsecontrolling layers (15c;15d) is respectively provided between one of the two intestinal collapse-controlling layers (12a;12b) and the corresponding gel-forming layer (13c;13d), and
two second medicine-containing layers (16a;16b) containing a medicine, wherein each one of the two second medicine-containing layers (16a;16b) is respectively provided between one of the two intestinal collapse-controlling layers (12a;12b) and the corresponding intragastric collapse-controlling layer (15c;15d).

11. The orally-administered agent (1b;1c;1d;1e) as claimed in one of the foregoing claims, wherein the pitch p between the convex portions is in a range of 250 to 750 µm.

12. The orally-administered agent (1b;1c;1d;1e) as claimed in one of the foregoing claims, wherein a width w of each of the convex portions is in a range of 20 to 300 µm, preferably in a range of 50 to 250 µm.

13. The orally-administered agent (1b;1c;1d;1e) as claimed in one of the foregoing claims, wherein a height d of each of the convex portions is in a range of 10 to 5,000 µm, preferably in a range of 20 to 1,000 µm.

14. An orally-administered agent (1a) comprising:
a medicine-containing layer (11) containing a medicine and having surfaces;
two intestinal collapse-controlling layers (12a;12b) including an enteric material which is dissolved by being in contact with the intestinal body fluid, wherein the intestinal collapse-controlling layers (12a;12b) are provided directly or through an arbitrary layer on the surfaces of the medicine-containing layer (11), respectively, and each of the intestinal collapse-controlling layers (12a;12b) has a surface opposite to the medicine-containing layer (11); and
two gel-forming layers (13a;13b) containing a gel-forming agent that is swelled and gelatinized by absorbing water, wherein the gel-forming layers (13a;13b) are provided directly or through an arbitrary layer on the sides of the surfaces of the intestinal collapse-controlling layers (12a;1.2b), respectively;
two antiadhesive layers (14a;14b) including an antiadhesive agent as surface layers constituting surfaces of the orally-administered agent (1a),
wherein a viscosity at 37°C of an aqueous solution of 5 mass% of the antiadhesive agent measured with an E-type viscometer is 50 mPa·s or less;
wherein the antiadhesive layers (14a;14b) prevent the orally-administered agent (1a) from adhering to an inside wall of an oral cavity by dissolving to water.

15. The orally-administered agent (1a) as claimed in claim 14, wherein the antiadhesive agent includes a water-soluble polymer material selected from the group consisting of polyethylene glycol, polyvinyl alcohol, and hydroxypropyl cellulose, and a mass-average molecular weight of the water-soluble polymer material is in a range of 5000 to 150000.

16. The orally-administered agent (1a) as claimed in claim 14, wherein the enteric material contains an enteric polymer.

17. The orally-administered agent (1a) as claimed in claim 16, wherein the enteric polymer is an enteric acrylic acid-based copolymer selected from the group consisting of a methacrylic acid-methyl methacrylate copolymer, methacrylic acid-ethyl acrylate, a styrene-acrylic acid copolymer, a methyl acrylate-acrylic acid copolymer, a methyl acrylate-methacrylic acid copolymer, a buthyl acrylate- styrene-acrylic acid copolymer, and a methyl acrylate-methacrylic acid-octyl acrylate copolymer, or a cellulose derivative selected from the group consisting of hydroxy-propyl-methyl cellulose acetate succinate, hydroxy-propyl-methyl cellulose phthalate, hydroxy-propyl-methyl acetate maleate, hydroxy-methyl-ethyl cellulose phthalate, cellulose acetate phthalate, cellulose acetate succinate, cellulose acetate trimellitate, cellulose acetate maleate, cellulose benzoate phthalate, cellulose propionate phthalate, methyl cellulose phthalate, ethylhydroxy-ethyl cellulose phthalate, hydroxy-methyl cellulose ethylphthalate, and cellulose acetate phthalate.

18. The orally-administered agent (1a) as claimed in claim 14, wherein the gel-forming layers (13a;13b) contain an anionic polymer.

## Patentansprüche

1. Oral verabreichtes Mittel (1b; 1c; 1d; 1e), umfassend:
eine Medikament-enthaltende Schicht (11; 11a), die ein Medikament enthält und Oberflächen aufweist;
zwei Darmzerfall-regulierende Schichten (12a; 12b; 12c; 12d), die ein magensaftresistentes Material enthalten, das bei Kontakt mit dem Darm-Körperfluid gelöst wird, wobei die Darmzerfallregulierenden Schichten (12a; 12b; 12c; 12d) direkt oder über eine beliebige Schicht auf den Oberflächen der Medikament-enthaltenden Schicht (11; 11a) bereitgestellt sind und jede der Darmzerfall-regulierenden Schichten (12a; 12b; 12c; 12d) eine Oberfläche aufweist, die der Medikament-enthaltenden Schicht (11; 11a) gegenüber liegt; und
zwei Gel-bildende Schichten (13c; 13d; 13e; 13f), die ein Gel-bildendes Mittel enthalten, das durch Absorbieren von Wasser quillt und geliert, wobei die Gel-bildenden Schichten (13c; 13d; 13e; 13f) direkt oder über eine beliebige Schicht an den Seiten der Oberflächen der Darmzerfallregulierenden Schichten (12a; 12b; 12c; 12d) bereitgestellt sind,
wobei jede der Gel-bildenden Schichten (13c; 13d; 13e; 13f) eine Oberfläche aufweist, die mit einer Vielzahl von konvexen Abschnitten versehen ist, und der Abstand p zwischen den konvexen Abschnitten im Bereich von 100 bis 1.000 µm liegt und
wobei die Oberflächen der Gel-bildenden Schichten (13c; 13d; 13e; 13f) die Außenoberflächen des oral verabreichten Mittels (1b; 1c; 1d; 1e) definieren.

2. Oral verabreichtes Mittel (1b; 1c; 1d; 1e) gemäß Anspruch 1, wobei die Medikament-enthaltende Schicht (11; 11a) vollständig von den Darmzerfallregulierenden Schichten (12a; 12b; 12c; 12d) bedeckt ist.

3. Oral verabreichtes Mittel (1b; 1c; 1d; 1e) gemäß Anspruch 1, wobei das magensaftresistente Material ein magensaftresistentes Polymer enthält.

4. Oral verabreichtes Mittel (1b; 1c; 1d; 1e) gemäß Anspruch 3, wobei das magensaftresistente Polymer ein magensaftresistentes Acrylsäure-basiertes Copolymer ist, ausgewählt aus der Gruppe bestehend aus einem Methacrylsäure-MethylmethacrylatCopolymer, Methacrylsäure-Ethylacrylat, einem Styrol-Acrylsäure-Copolymer, einem MethylacrylatAcrylsäure-Copolymer, einem MethylacrylatMethacrylsäure-Copolymer, einem ButylacrylatStyrol-Acrylsäure-Copolymer und einem Methylacrylat-Methacrylsäure-Octylacrylat-Copolymer, oder einem Cellulosederivat, ausgewählt aus der Gruppe bestehend aus Hydroxypropylmethylcelluloseacetatsuccinat, Hydroxypropylmethylcellulosephthalat, Hydroxypropylmethylacetatmaleat, Hydroxymethylethylcellulosephthalat, Celluloseacetatphthalat, Celluloseacetatsuccinat, Celluloseacetattrimellitat, Celluloseacetatmaleat, Cellulosebenzoatphthalat, Cellulosepropionatphthalat, Methylcellulosephthalat, Ethylhydroxyethylcellulosephthalat, Hydroxymethylcelluloseethylphthalat und Celluloseacetatphthalat.

5. Oral verabreichtes Mittel (1b; 1c; 1d; 1e) gemäß Anspruch 1, wobei die Gel-bildenden Schichten ein anionisches Polymer enthalten.

6. Oral verabreichtes Mittel (1b; 1c; 1d; 1e) gemäß Anspruch 1, wobei die Gel-bildenden Schichten (13c; 13d; 13e; 13f) einen Wasserabsorptionsförderer zum Fördern der Wasserabsorption der Gel-bildenden Schichten (13c; 13d; 13e; 13f) enthalten.

7. Oral verabreichtes Mittel (1b; 1c; 1d; 1e) gemäß Anspruch 6, wobei, wenn eine wässrige Lösung von 5 Masse-% des Wasserabsorptionsförderers hergestellt wird, die mittels eines E-Typ-Viskosimeters gemessene Viskosität der wässrigen Lösung bei 37 °C im Bereich von 0,3 bis 5,0 mPa·s liegt.

8. Oral verabreichtes Mittel (1b; 1c; 1d; 1e) gemäß Anspruch 1, wobei die Darmzerfall-regulierenden Schichten (12a; 12b; 12c; 12d) ein Medikament enthalten, das von dem in der Medikament-enthaltenden Schicht (11; 11a) enthaltenden Medikament verschieden ist.

9. Oral verabreichtes Mittel (1c) gemäß Anspruch 1, ferner umfassend zwei Magenzerfall-regulierende Schichten (15a; 15b), die ein magenlösliches Material enthalten, das bei Kontakt mit Magensaft gelöst wird, wobei jede der beiden Magenzerfall-regulierenden Schichten (15a; 15b) jeweils zwischen einer der beiden Darmzerfall-regulierenden Schichten (12a; 12b) und der entsprechenden Gel-bildenden Schicht (13c; 13d) angeordnet ist.

10. Oral verabreichtes Mittel (1d) gemäß Anspruch 1, ferner umfassend
zwei Magenzerfall-regulierende Schichten (15c; 15d), die ein magenlösliches Material enthalten, das bei Kontakt mit Magensaft gelöst wird, wobei jede der beiden Magenzerfall-regulierenden Schichten (15c; 15d) jeweils zwischen einer der beiden Darmzerfall-regulierenden Schichten (12a; 12b) und der entsprechenden Gel-bildenden Schicht (13c; 13d) angeordnet ist, und
zwei zweite Medikament-enthaltende Schichten (16a; 16b), die ein Medikament enthalten, wobei jede der beiden zweiten Medikament-enthaltenden Schichten (16a; 16b) jeweils zwischen einer der beiden Darmzerfall-regulierenden Schichten (12a; 12b) und der entsprechenden Magenzerfall-regulierenden Schicht (15c; 15d) angeordnet ist.

11. Oral verabreichtes Mittel (1b; 1c; 1d, 1e) gemäß einem der vorstehenden Ansprüche, wobei der Abstand p zwischen den konvexen Abschnitten im Bereich von 250 bis 750 µm liegt.

12. Oral verabreichtes Mittel (1b; 1c; 1d; 1e) gemäß einem der vorstehenden Ansprüche, wobei die Breite w jedes der konvexen Abschnitte im Bereich von 20 bis 300 µm liegt, vorzugsweise im Bereich von 50 bis 250 µm.

13. Oral verabreichtes Mittel (1b; 1c; 1d; 1e) gemäß einem der vorstehenden Ansprüche, wobei die Höhe d jedes der konvexen Abschnitte im Bereich von 10 bis 5.000 µm liegt, vorzugsweise im Bereich von 20 bis 1.000 µm.

14. Oral verabreichtes Mittel (1a), umfassend:
eine Medikament-enthaltende Schicht (11), die ein Medikament enthält und Oberflächen aufweist;
zwei Darmzerfall-regulierende Schichten (12a; 12b), die ein magensaftresistentes Material enthalten, das bei Kontakt mit dem Darm-Körperfluid gelöst wird, wobei die Darmzerfall-regulierenden Schichten (12a; 12b) direkt oder über eine beliebige Schicht auf den Oberflächen der Medikament-enthaltenden Schicht (11) bereitgestellt sind und jede der Darmzerfall-regulierenden Schichten (12a; 12b) eine Oberfläche aufweist, die der Medikament-enthaltenden Schicht (11) gegenüber liegt; und
zwei Gel-bildende Schichten (13a; 13b), die ein Gel-bildendes Mittel enthalten, das durch Absorbieren von Wasser quillt und geliert, wobei die Gel-bildenden Schichten (13a; 13b) direkt oder über eine beliebige Schicht an den Seiten der Oberflächen der Darmzerfall-regulierenden Schichten (12a; 12b) bereitgestellt sind,
zwei Antihaftschichten (14a; 14b), die ein Antihaftmittel als Oberflächenschichten, die Oberflächen des oral verabreichten Mittels (1a) bilden, enthalten,
wobei die mittels eines E-Typ-Viskosimeters gemessene Viskosität einer wässrigen Lösung von 5 Masse-% des Antihaftmittels bei 37 °C 50 mPa·s oder weniger beträgt;
wobei die Antihaftschichten (14a; 14b) durch Lösen an Wasser verhindern, dass das oral verabreichte Mittel (1a) an einer Innenwand einer Mundhöhle haftet.

15. Oral verabreichtes Mittel (1a) gemäß Anspruch 14, wobei das Antihaftmittel ein wasserlösliches Polymermaterial enthält, ausgewählt aus der Gruppe bestehend aus Polyethylenglycol, Polyvinylalkohol und Hydroxypropylcellulose, und das massengemittelte Molekulargewicht des wasserlöslichen Polymermaterials in Bereich von 5000 bis 150000 liegt.

16. Oral verabreichtes Mittel (1a) gemäß Anspruch 14, wobei das magensaftresistente Material ein magensaftresistentes Polymer enthält.

17. Oral verabreichtes Mittel (1a) gemäß Anspruch 16, wobei das magensaftresistente Polymer ein magensaftresistentes Acrylsäure-basiertes Copolymer ist, ausgewählt aus der Gruppe bestehend aus einem Methacrylsäure-Methylmethacrylat-Copolymer, Methacrylsäure-Ethylacrylat, einem Styrol-Acrylsäure-Copolymer, einem Methylacrylat-Acrylsäure-Copolymer, einem Methylacrylat-Methacrylsäure-Copolymer, einem Butylacrylat-Styrol-Acrylsäure-Copolymer und einem Methylac-rylat-Methacrylsäure-Octylacrylat-Copolymer, oder einem Cellulosederivat, ausgewählt aus der Gruppe bestehend aus Hydroxypropylmethylcelluloseacetatsuccinat, Hydroxypropylmethylcellulosephthalat, Hydroxypropylmethylacetatmaleat, Hydroxymethylethylcellulosephthalat, Celluloseacetatphthalat, Celluloseacetatsuccinat, Celluloseacetattrimellitat, Celluloseacetatmaleat, Cellulosebenzoatphthalat, Cellulosepropionatphthalat, Methylcellulosephthalat, Ethylhydroxyethylcellulosephthalat, Hydroxymethylcelluloseethylphthalat und Celluloseacetatphthalat.

18. Oral verabreichtes Mittel (1a) gemäß Anspruch 14, wobei die Gel-bildenden Schichten (13a; 13b) ein anionisches Polymer enthalten.

## Revendications

1. Agent administré par voie orale (1b ; 1c ; 1d; 1e) comprenant :
une couche contenant un médicament (11 ; 11a) qui contient un médicament et qui présente des surfaces ;
deux couches de contrôle de la dégradation intestinale (12a ; 12b ; 12c ; 12d) comprenant un matériau gastro-résistant qui est dissout en étant en contact avec le fluide corporel intestinal, dans lequel les couches de contrôle de la dégradation intestinale (12a ; 12b ; 12c ; 12d) sont fournies directement ou par l'intermédiaire d'une couche arbitraire sur les surfaces de la couche contenant un médicament (11 ; 11a), respectivement, et chacune des couches de contrôle de la dégradation intestinale (12a ; 12b ; 12c ; 12d) présente une surface opposée à la couche contenant un médicament (11 ; 11a) ; et
deux couches de gélifiant (13c ; 13d ; 13e ; 13f) contenant un agent gélifiant qui se gonfle et se gélifie en absorbant de l'eau, dans lequel les couches de gélifiant (13c ; 13d ; 13e ; 13f) sont prévues directement ou par l'intermédiaire d'une couche arbitraire sur les côtés des surfaces des couches de contrôle de la dégradation intestinale (12a ; 12b ; 12c ; 12d), respectivement,
dans lequel chacune des couches de gélifiant (13c ; 13d ; 13e ; 13f) présente une surface munie d'une pluralité de parties convexes et un pas p entre les parties convexes se situe dans une plage allant de 100 à 1000 µm, et dans lequel les surfaces des couches de gélifiant (13c ; 13d ; 13e ; 13f) définissent des surfaces extérieures de l'agent administré par voie orale (1b ; 1c ; 1d ; le).

2. Agent administré par voie orale (1b ; 1c ; 1d ; 1e) selon la revendication 1, dans lequel la couche contenant un médicament (11 ; 11 a) est complètement recouverte par les couches de contrôle de la dégradation intestinale (12a ; 12b ; 12c ; 12d).

3. Agent administré par voie orale (1b ; 1c ; 1d ; 1e) selon la revendication 1, dans lequel le matériau gastro-résistant contient un polymère gastro-résistant.

4. Agent administré par voie orale (1b ; 1c ; 1d ; 1e) selon la revendication 3, dans lequel le polymère gastro-résistant est un copolymère à base d'acide acrylique gastro-résistant choisi dans le groupe constitué d'un copolymère d'acide méthacrylique - méthacrylate de méthyle, acide méthacrylique - acrylate d'éthyle, un copolymère de styrène - acide acrylique, un copolymère d'acrylate de méthyle - acide acrylique, un copolymère d'acrylate de méthyle - acide méthacrylique, un copolymère d'acrylate de butyle - styrène - acide acrylique, et un copolymère d'acrylate de méthyle - acide méthacrylique - acrylate d'octyle, ou un dérivé de cellulose choisi dans le groupe constitué de succinate d'acétate d'Hydroxypropylméthylcellulose, de phthalate d'hydroxypropylméthylcellulose, de maléate d'Hydroxypropylméthylacétate, de phtalate d'hydroxyméthyléthylcellulose, de phtalate d'acétate de cellulose, de succinate d'acétate de cellulose, de trimellitate d'acétate de cellulose, de maléate d'acétate de cellulose, de phtalate de benzoate de cellulose, de phtalate de propionate de cellulose, de phtalate de méthylcellulose, de phtalate d'éthylhydroxyéthylcellulose, de phtalate d'ethyl d'hydroxyméthylcellulose, et de phtalate d'acétate de cellulose.

5. Agent administré par voie orale (1b ; 1c ; 1d ; 1e) selon la revendication 1, dans lequel les couches de gélifiant contiennent un polymère anionique.

6. Agent administré par voie orale (1b ; 1c ; 1d ; 1e) selon la revendication 1, dans lequel les couches de gélifiant (13c ; 13d ; 13e ; 13f) contiennent un promoteur d'absorption de l'eau favorisant l'absorption de l'eau des couches de gélifiant (13c ; 13d; 13e ; 13f).

7. Agent administré par voie orale (1b ; 1c ; 1d ; 1e) selon la revendication 6, dans lequel, lorsqu'une solution aqueuse de 5% en masse du promoteur d'absorption de l'eau est préparée, une viscosité à 37° C de la solution aqueuse mesurée avec un viscosimètre de type E se situe dans une plage allant de 0,3 à 5,0 mPa·s.

8. Agent administré par voie orale (1b ; 1c ; 1d ; 1e) selon la revendication 1, dans lequel les couches de contrôle de la dégradation intestinale (12a ; 12b ; 12c ; 12d) contiennent un médicament qui est différent du médicament contenu dans couche contenant un médicament (11 ; 11a).

9. Agent administré par voie orale (1c) selon la revendication 1, comprenant en outre deux couches de contrôle de la dégradation intragastrique (15a ; 15b) contenant un matériau soluble dans l'estomac qui est dissous en étant en contact avec le suc gastrique, dans lequel chacune des deux couches de contrôle de la dégradation intragastrique (15a ; 15b) est respectivement prévue entre l'une des deux couches de contrôle de la dégradation intestinale (12a ; 12b) et la couche de gélifiant (13c ; 13d) correspondante.

10. Agent administré par voie orale (3d) selon la revendication 1, comprenant en outre :
deux couches de contrôle de la dégradation intragastrique (15c ; 15d) contenant un matériau soluble dans l'estomac qui se dissout en étant en contact avec le suc gastrique, dans lequel chacune des deux couches de contrôle de la dégradation intragastrique (15c ; 16d) est respectivement prévue entre l'une des deux couches de contrôle de la dégradation intestinale (12a ; 12b) et la couche de gélifiant (13c ; 13d) correspondante, et
deux deuxièmes couches contenant un médicament (16a ; 16b) qui contiennent un médicament, dans lequel chacune des deux deuxièmes couches contenant un médicament (16a ; 16b) est respectivement prévue entre l'une des deux couches de contrôle de la dégradation intestinale (12a ; 12b) et la couche de contrôle de la dégradation intragastrique (15c ; 15d) correspondante.

11. Agent administré par voie orale (16 ; 1c ; 1d ; 1e) selon l'une quelconque des revendications précédentes, dans lequel le pas p entre les parties convexes se situe dans une plage allant de 250 à 750 µm.

12. Agent administré par voie orale (1b ; 1c ; 1d ; 1e) selon l'une quelconque des revendications précédentes, dans lequel une largeur w de chacune des parties convexes se situe dans une plage allant de 20 à 300 µm, de préférence dans une plage allant de 50 à 250 µm.

13. Agent administré par voie orale (1b ; 1c ; 1d ; 1e) selon l'une quelconque des revendications précédentes, dans lequel une hauteur d de chacune des parties convexes se situe dans une plage allant de 10 à 5000 µm de préférence dans une plage allant de 20 à 1000 µm.

14. Agent administré par voie orale (1a) comprenant :
une couche contenant un médicament (11) qui contient un médicament et qui présente des surfaces ;
deux couches de contrôle de la dégradation intestinale (12a ; 12b) comprenant un matériau gastro-résistant qui est dissout en étant en contact avec le fluide corporel intestinal, dans lequel les couches de contrôle de la dégradation intestinale (12a ; 12b) sont prévues directement ou par l'intermédiaire d'une couche arbitraire sur les surfaces de la couche contenant un médicament (11), respectivement, et chacune des couches de contrôle de la dégradation intestinale (12a ; 12b) présente une surface opposée à la couche contenant un médicament (11) ; et
deux couches de gélifiant (13a ; 13b) contenant un agent gélifiant qui se gonfle et se gélifie en absorbant de l'eau, dans lequel les couches de gélifiant (13a ; 13b) sont prévues directement ou par l'intermédiaire d'une couche arbitraire sur les côtés des surfaces des couches de contrôle de la dégradation intestinale (12a ; 12b), respectivement ;
deux couches anti- adhésives (14a ; 14b), comprenant un agent anti- adhésif en tant que couches de surface constituant des surfaces de l'agent administré par voie orale (1a),
dans lequel une viscosité à 37 °C d'une solution aqueuse de 5% en masse de l'agent anti-adhésif mesurée avec un viscosimètre de type E est de 50 mPa·s s ou moins ;
dans lequel les couches anti-adhésives (14a ; 14b) empêchent l'agent administré par voie orale (la) d'adhérer à une paroi intérieure d'une cavité buccale en se dissolvant dans l'eau.

15. Agent administré par voie orale (1a) selon la revendication 14, dans lequel l'agent anti- adhésif comprend un matériau polymère soluble dans l'eau choisi dans le groupe constitué par le polyéthylène glycol, l'alcool polyvinylique, et l'hydroxypropylcellulose, et un poids moléculaire moyen en masse du matériau polymère soluble dans l'eau se situe dans une plage allant de 5000 à 150000.

16. Agent administré par voie orale (1a) selon revendication 14, dans lequel le matériau gastro-résistant contient un polymère gastro-résistant.

17. Agent administré par voie orale (1a) selon la revendication 16, dans lequel dans lequel le polymère gastro-résistant est un copolymère à base d'acide acrylique gastro-résistant choisi dans le groupe constitué d'un copolymère d'acide méthacrylique - méthacrylate de méthyle, acide méthacrylique - acrylate d'éthyle, un copolymère de styrène - acide acrylique, un copolymère d'acrylate de méthyle - acide acrylique, un copolymère d'acrylate de méthyle - acide méthacrylique, un copolymère d'acrylate de butyle - styrène - acide acrylique, et un copolymère d'acrylate de méthyle - acide méthacrylique - acrylate d'octyle, ou un dérivé de cellulose choisi dans le groupe constitué de succinate d'acétate d'Hydroxypropylméthylcellulose, de phthalate d'hydroxypropylméthylcellulose, de maléate d'Hydroxypropylméthylacétate, de phtalate d'hydroxyméthyléthylcellulose, de phtalate d'acétate de cellulose, de succinate d'acétate de cellulose, de trimellitate d'acétate de cellulose, de maléate d'acétate de cellulose, de phtalate de benzoate de cellulose, de phtalate de propionate de cellulose, de phtalate de méthylcellulose, de phtalate d'éthylhydroxyéthylcellulose, de phtalate d'ethyl d'hydroxyméthylcellulose, et de phtalate d'acétate de cellulose.

18. Agent administré par voie orale (1a) selon la revendication 14, dans lequel les couches de gélifiant (13a ; 13b) contiennent un polymère anionique.
